# EUROPEAN PATENT SPECIFICATION

(11) **EP 3 941 398 B1**
(45) Date of publication and mention of the grant of the patent: **05.11.2025**
(21) Application number: 20773693.5
(22) Date of filing: 17.03.2020
(51) Int. Cl.: A61F 2/04, A61F 2/844, A61F 2/848, A61F 2/06, A61F 2/966

(54) **STENT WITH ANTI-MIGRATION DEVICES**
STENT MIT MIGRATIONSSCHUTZVORRICHTUNGEN
ENDOPROTHÈSE AVEC DISPOSITIFS ANTI-MIGRATION

(30) Priority: 19.03.2019 US 201916357812
(43) Date of publication of application: 26.01.2022
(73) Proprietor: Q3 Medical Devices Limited, Dublin 2 (IE); Mangiardi, Eric K., Charlotte, NC 28211 (US)
(72) Inventor: MANGIARDI, Eric, K., Charlotte, NC 28211 (US)
(74) Representative: Brand Murray Fuller LLP
(86) International application number: PCT/US2020/023172
(87) International publication number: WO 2020/190953

(56) References cited:
- WO-A1-2017/209798
- US-A1- 2003 120 338
- US-A1- 2008 045 982
- US-A1- 2009 005 760
- US-A1- 2017 071 769
- US-A1- 2017 128 188
- US-B2- 8 394 139

## Description

This application claims priority to U.S. Patent Application Serial No.16/357,812, filed March 19, 2019.

### FIELD

The present application generally relates to medical devices and, in particular, to a in vivo supporting devices such as stents.

### BACKGROUND

In medical terms, a stent is a man-made "tube" inserted into a natural passage or conduit in the body to prevent, or counteract, a disease-induced, localized flow constriction. The term may also refer to a tube used to temporarily hold such a natural conduit open to allow access for surgery. Stents include vascular and non-vascular stents. Vascular stents are designed for applications in the vascular system, such as arteries and veins. Non-vascular stents are used in other body lumens such as biliary, colorectal, esophageal, ureteral and urethral tract, and upper airway.

Stents are available in permanent and temporary varieties. Stent duration is heavily influenced by the construction material. For example, metal stents typically have a much longer use life than plastic stents. The stent body typically has a central lumen that allows blood or other body fluid to flow through the stent. A common problem with the current stents is that they routinely migrate and clog, thus requiring additional procedures for extraction and/or replacement. There exists a need for improved stents that are easy to make and safe to use.

In chronic pancreatitis, a fibrotic duct stricture is a common complication and a therapeutic challenge. Drainage of an obstructed duct becomes mandatory because the intraductal pressure created by the stricture causes severe pain. At present, these fibrotic strictures are endoscopically treated by the sequential placement of multiple plastic stents for a period of six to twelve months with stent exchanges approximately every three months. These procedures are expensive and can increase risks for patients suffering from comorbidities. Therefore, there is a need for a biodegradable stent device, particularly for biliary or pancreatic placements, that has superior properties for supporting a vessel, duct or lumen and optimizing the flow of bodily fluids through.

However, a common problem with in vivo supporting devices, such as stents, is that they may move from the treatment site after implantation. Therefore, a need exists for improved in vivo supporting devices that are biodegradable in a controlled manner so that the supporting devices remain in their intended location for the intended period of time.

WO 2017/209798 relates to a stent with two open spiral channels formed on the exterior surface of the body and a central lumen open for the passage of a guide wire.

### SUMMARY

The present invention relates to a stent comprising: an elongated body composed of a biodegradable material having a proximal end, a distal end, at least one open spiral channel formed on the exterior surface of said body to provide fluid communication between said proximal end and said distal end, a central lumen open at the proximal and distal ends of the stent for the passage of a guide wire, wherein said stent further comprises at least two anti-migration tails extending beyond at least one end of the elongated body, wherein the at least two anti-migration tails extending from at least one end of the elongated body form a Y-shape with the elongated body, and wherein said anti-migration tails are flat-shaped at their ends.

Also disclosed herein but not claimed is a method of controlling fluid flow through a stent in a subject in need thereof, comprising: establishing an entry portal into a vessel, duct or lumen contiguous with a target site for stent placement, advancing a guide wire through the entry portal and said vessel, duct or lumen contiguous to said target site, advancing said stent along said guide wire to said target site, said stent comprising an elongated body composed of a bioabsorbable polymer having a proximal end, a distal end, at least one open spiral channel formed on the exterior surface of said body to provide fluid communication between said proximal end and said distal end and a central lumen open at the proximal and distal ends of the stent for the passage of a guide wire, withdrawing the guide wire, and controlling the fluid flow through the spiral channels by setting number of twists of the stent per inch.

Further disclosed herein but not claimed is a method of immobilizing a stent in a subject in need thereof, comprising: establishing an entry portal into a vessel, duct or lumen contiguous with a target site for stent placement, advancing a guide wire through the entry portal and said vessel, duct or lumen contiguous to said target site, advancing said stent along said guide wire to said target site, said stent comprising an elongated body composed of a bioabsorbable polymer having a proximal end, a distal end, at least one open spiral channel formed on the exterior surface of said body to provide fluid communication between said proximal end and said distal end and a central lumen open at the proximal and distal ends of the stent for the passage of a guide wire, immobilizing the stent at the target site by using an anti-migration device extending from at least one end of the elongated body, and withdrawing the guide wire.

### BRIEF DESCRIPTION OF THE DRAWINGS

The present application can be better understood by reference to the following drawings, wherein like references numerals represent like elements. The drawings are merely exemplary to illustrate certain features that may be used singularly or in combination with other features and the present application should not be limited to the embodiments shown.
FIG. 1 A is a diagram showing a stent disclosed herein. FIG. IB is a see-through illustration of FIG. 1A.
FIG. 2 is a diagram showing a stent disclosed herein with a sinusoidal shaped stent body.
FIG. 3 is a diagram showing an assembly of a stent disclosed herein with a guide wire and a pusher tube.
FIGS. 4A and 4B are diagrams showing two engagement mechanisms among the stent, the guide wire and the pusher tube.
FIGS. 5 A and 5B show an expandable stent disclosed herein.
FIGS. 6A and 6B show another expandable stent disclosed herein.
FIGS. 7A and 7B show another expandable stent disclosed herein.
FIGS. 8A-8F show various expandable stents disclosed herein.
FIGS. 9A-9C show several stents with an outer frame disclosed herein.
FIG. 10 shows another stent with sinusoidal channels of varying pitches disclosed herein.
FIGS. 11 A-11B show another stent of the present disclosure.
FIG. 12 shows another stent of the present disclosure.
FIG. 13 shows the cross section of a stent of the present disclosure.
FIG. 14 shows the cross section of another stent of the present disclosure.
FIG. 15A shows another stent of the present disclosure.
FIG. 15B shows an embodiment of a stent of the present invention.
FIG. 16 shows another stent of the present disclosure.
FIG. 17 shows another stent of the present disclosure.
FIG. 18 shows another stent of the present disclosure.
FIGS. 19A-G show another embodiment of a stent of the present invention.
FIGS. 20A-D show another stent of the present disclosure.
FIGS. 21A-H show another stent of the present disclosure.
FIGS. 22A-B show an alternative feature for the stents of FIGS 19A-21H.
FIG. 23 shows the results of a simulated flow test involving a stent of the present disclosure.
FIG. 24 shows the results of a simulated migration resistance test involving a stent of the present disclosure.
FIG. 25 shows the results of a crush resistance test involving a stent of the present disclosure.

### DETAILED DESCRIPTION

The practice of the subject matter of the present application will employ, unless otherwise indicated, conventional medical devices and methods within the skill of the art. Such techniques are explained fully in the literature.

The present application relates to a stent that contains an elongated stent body having a proximal end, a distal end, and two open spiral channels formed on the exterior surface of the elongated stent body to provide fluid communication from the proximal end to the distal end, a central lumen open at the proximal and distal ends of the stent for the passage of a guide wire , and wherein said stent further comprises at least two anti-migration tails extending beyond at least one end of the elongated body, wherein the at least two anti-migration tails extending from at least one end of the elongated body form a Y-shape with the elongated body, and wherein said anti-migration tails are flat-shaped at their ends..

As used herein, the term "stent" refers to a device which is implanted within a bodily lumen to hold open the lumen or to reinforce a small segment of the lumen. Stents can be used for treating obstructed vessels, biliary ducts, pancreatic ducts, ureters, or other obstructed lumens, fractured canals, bones with hollow centers and/or for delivering various drugs through controlled release to the particular lumen of interest.

The stent of the present application comprises a biodegradable material. The term "biodegradable" refers to materials that are capable of being completely degraded and/or eroded when exposed to bodily fluids such as blood and can be gradually resorbed, absorbed and/or eliminated by the body. "Biodegradable" is intended to broadly include biologically erodable, bioabsorbable, and bioresorbable materials such as alloys and polymers that are broken down and/or eliminated by the body. In some embodiments, at least a portion of the surface of the stent of the present application is polymeric. The stents of the present application are fabricated partially or completely from a biodegradable, or bioabsorbable polymer. In some embodiments, a polymer-fabricated stent may serve as a substrate for a polymer-based coating. The polymer-based coating may contain, for example, an active agent or drug for local administration at a diseased site. In some embodiments, an active agent or a drug is incorporated into a body of a polymer-fabricated stent.

The open channel should be large enough to allow unobstructed or normal flow of various body fluids such as blood, bile or urine or other luminal material/liquids on the outer aspect of the stent. The open channel may have a cross section area that is of any shape or depth. The channel could be V shapes, U shaped, or with a rising or falling pitch, of an even depth or one that is of varying widths, depths, varying and circumferential rotations changing at various points over the length of the device. The channel can be a straight channel or a spiral channel. Multiple channels may be formed on the exterior surface or the inner surface of the elongated stent body. The channel(s) may also be designed with a geometry that would help the stent to remain in place. A double-channel helical twist design refers to a stent with two open spiral channels on the exterior surface of the stent body to provide fluid communication between the opposing ends of the stent body (proximal end and distal end). The stent of the present application in a preferred embodiment has a double channel helical twist design.

The shape, length and diameter of the stent body are application dependent. The elongated stent body can be straight or curved or in the shape of multiply connected and angulated curves. Each type of stent is designed to fit within a specific part of the anatomy. Therefore, the shape, length, and diameter of stents differ by type to accommodate and support different sized lumens and different clinical needs. For example, each major stent application, such as vascular, pancreatic, ureteral, or metacarpal canal, other hollow bone structures and other stent, requires a different diameter and shape to enable placement, to remain in place after placement, to stabilize and support the anatomy it is placed in, and to allow conformance to the normal anatomy. As used herein, the diameter of a stent refers to the width across the shaft of the stent body, which is also referred to as the "major diameter." In one embodiment, the stent has a uniform diameter. In another embodiment, the stent has a variable diameter. In one embodiment, the diameter at the distal end is smaller than the diameter at the proximal end. In another embodiment, the diameter at the proximal end is smaller than the diameter at the distal end. In yet another embodiment, the diameters at the distal end and the proximal end are both smaller than the diameter at the middle section of the stent.

The stent body includes a center lumen to accommodate a guide wire. This center lumen may provide additionally flow throughput after the removal of guide wire.

The present application relates to a stent comprising an elongated body having a proximal end, a distal end, at least one open spiral channel formed on the exterior surface of said body to provide fluid communication between said proximal end and said distal end, and a central lumen open at the proximal and distal ends of the stent for the passage of a guide wire.

In some embodiments, the body is composed of a bioabsorbable polymer.

In other embodiments, the at least one open spiral channel has a rotation rate of between about 1.5 and 2.5 twists per inch.

In still other embodiments, the at least one open spiral channel has a rotation rate of at least about 2 twists per inch.

In some embodiments, the stent comprises two open spiral channels formed on the exterior surface of said body. In some further embodiments, the channels are on opposite sides on the exterior surface of said body.

In some embodiments, the body further comprises an anti-migration device.

In other embodiments, the body further comprises a biological agent. In some further embodiments, the biological agent is selected from the group consisting of chemotherapeutic agents, antimicrobial agents and gene transfer agents.

In particular an embodiment, the stent has a pre-implantation diameter Dₚᵣₑ and is *in situ* expandable upon absorption of a body fluid to a post-implantation diameter Dₚₒₛₜ, wherein Dₚₒₛₜ is greater than Dₚᵣₑ .

In some embodiments, the stent comprises a radio-opaque substance.

The stent may comprise an elongated body composed of a bioabsorbable polymer and having a proximal end, a distal end, at least one open spiral channel formed on the exterior surface of said body to provide fluid communication between said proximal end and said distal end, wherein said at least one open spiral channel has a rotation rate of at least 1 twist per inch, and a central lumen open at the proximal and distal ends of the stent for the passage of a guide wire.

In some embodiments, the bioabsorbable polymer comprises PEG and p-dioxanone.

In other embodiments, the bioabsorbable polymer comprises PPDO.

In still other embodiments, the bioabsorbable polymer comprises PLA, trimethylene carbonate and caprolactone.

In some embodiments, the at least one open spiral channel has a rotation rate of at least about 2 twists per inch.

In particular embodiments, the stent comprises two open spiral channels formed on the exterior surface of said body. In some further embodiments, the channels are on opposite sides on the exterior surface of said body.

The stent may comprise an elongated body having a proximal end, a distal end, at least one open spiral channel formed on the exterior surface of said body to provide fluid communication between said proximal end and said distal end, wherein said at least one open spiral channel has a rotation rate of between about 1.5 and 3.5 twists per inch, and a central lumen open at the proximal and distal ends of the stent for the passage of a guide wire.

In some embodiments, the stent comprises two open spiral channels formed on the exterior surface of said body. In some further embodiments, the channels are on opposite sides on the exterior surface of said body.

The stent may comprise an elongated body composed of a bioabsorbable polymer and having a proximal end, a distal end, a pair of open spiral channels formed on the exterior surface of said body to provide fluid communication between said proximal end and said distal end, wherein said spiral channels have a rotation rate of at least about 1 twist per inch, and a central lumen open at the proximal and distal ends of the stent for the passage of a guide wire.

In some embodiments, the channels are on opposite sides on the exterior surface of said body.

Disclosed herein, but not claimed, is a method of emplacing a stent in a subject in need thereof, comprising: establishing an entry portal into a vessel, duct or lumen contiguous with a target site for stent placement, advancing a guide wire through the entry portal and said vessel, duct or lumen contiguous to said target site, and advancing the stent along said guide wire to the target site. The stent comprises an elongated body having a proximal end, a distal end, two open spiral channels formed on the exterior surface of said body to provide fluid communication between said proximal end and said distal end and a central lumen open at the proximal and distal ends of the stent for the passage of a guide wire. The method further comprises the step of withdrawing the guide wire.

Also disclosed herein, but not claimed, is a kit for stent placement. The kit comprises a stent comprising an elongated body having a proximal end, a distal end, two open spiral channels formed on the exterior surface of said body to provide fluid communication between said proximal end and said distal end and a central lumen open at the proximal and distal ends of the stent for the passage of a guide wire. The kit also comprises a guide wire.

In one embodiment, the stent is naturally formed by braiding multiple filaments together. In another embodiment, the stent is made with a center rod/hub/cam having one or more sinusoidal channels running through the exterior surface of the center rod, similar to that of a drill bit.

The stent of the present application can be expandable. In one embodiment, the stent is of two different diametrical dimensions due to radial deformation of its elastic elements. Before being positioned at the place of reconstruction, the stent is deformed/compressed/folded so as to minimize its diametrical dimension. Then the stent is placed, in the deformed state, inside a transporting means by arranging it on a special setting bulb. Once the stent has been transported to the place of reconstruction, the setting bulb is expanded so that the stent diameter is maximized. In another embodiment, the stent has a plurality of flexible or foldable channel walls or leaflets extending from the center rod/hub/cam. The channel walls or leaflets are kept in a folded position during the delivery process and are released only at the treatment site.

In one embodiment, the stent is delivered to the treatment site in a body lumen with a pusher rod that pushes the stent through a body channel into place. The pusher rod travels over a guide wire. The pusher rod is designed in such a way to attach to the ends of the stent to assist with directing the delivery. In one embodiment, the pusher rod interlocks with the proximal end of the stent in a male/female fashion, much the same way a wrench fits over a nut.

The stent of the present application can replace plastic and biliary and/or pancreatic stents with the additional benefit of being biodegradable, which eliminates the use of endoscopic retrograde cholangiopancreatography (ERCP) for subsequent stent removal. In particular embodiments, the stent of the present application may have one of three or more degradation profiles, including a fast degradation profile (minimal strength retention of 12 days), a medium degradation profile (minimal strength retention of 25 days) and a slow degrading profile (minimal strength retention of 12 weeks). The strength retention is defined by the presence of at least 10% of the initial strength parameter (e.g. the stent remains intact with no breaks, tested in a simulated degradation model).

In some embodiments, the stent with a fast degradation profile contains a polymer of 20% PEG and 80% p-dioxanone and is impregnated with BaSCri. In one embodiment, the stent with a fast degradation profile contains by weight 16.8% PEG, 67.2% p-dioxanone (in the form of a 20%/80% PEG/p-dioxanone coploymer) and 16% BaS04 (barium sulfate), In some embodiments, the stent with a medium degradation profile contains a polymer of 100% poly(para-dioxanone) and is impregnated with BaSCri. In one embodiment, the stent with a medium degradation profile contains by weight 84% Poly(para- dioxanone) and 16% BaS04. In some embodiments, the stent with a slow degradation profile contains a copolymer of 74% lactide, 15% trimethylene carbonate, 11% caprolactone and is impregnated with BaSCri. In one embodiment, the stent with a slow degradation profile contains by weight 62.16% lactide, 12.6% trimethylene carbonate, 9.24% caprolactone (in the form of a 74%/ 15 %/ 11% coploymer) and 16% BaS04.

In some embodiments, the stent of the present application experiences initial surface degradation upon implantation, which allows for bile cleansing (one of ordinary skill will understand that certain polymers will degrade by surface erosion before bulk degradation, no special coating or composition is required). The stent of the present application may also use a double-channel helical twist design to allow bile flow on the outside of the stent. In comparison to other stents, the stent of the present application provides better simulated flow rates, better simulated migration resistance and better crush resistance.

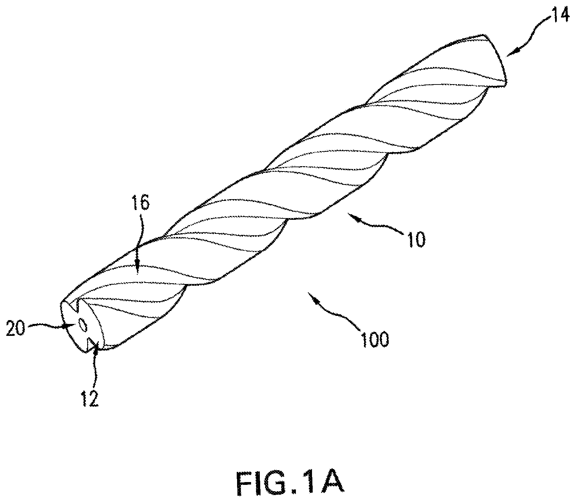

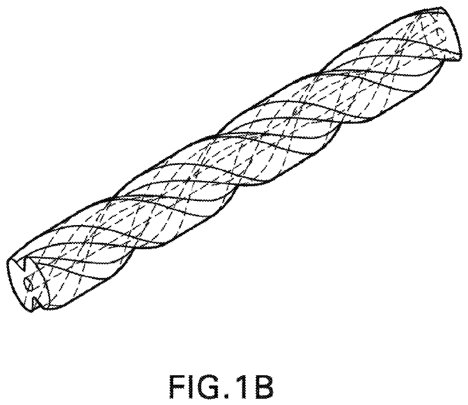

FIG. 1 A is a diagram showing a stent disclosed herein. In this instance, stent 100 has an elongated body 10 with a proximal end 12 and a distal end 14. Two sinusoidal channels 16 are formed on the exterior surface of the elongated body 10, extending from the proximal end 12 to the distal end 14 in a fashion similar to the grooves on a drill head. The channels may have beveled edges to facilitate fluid flow inside the channels. The channels can be of varying depths and lengths. The ends of the stent body can be of various shapes including conical shape. FIG. IB is a see through drawing of FIG. 1A. The two-channel design allows for two channels on the exterior surface of the stent to run in parallel from one end to the other or to criss-cross to allow for increased fluid flow as well as the ability to increase side branch flow of the main stented channel.

A center lumen 20 allows the stent 100 to slide into the place of implantation through a guide wire.

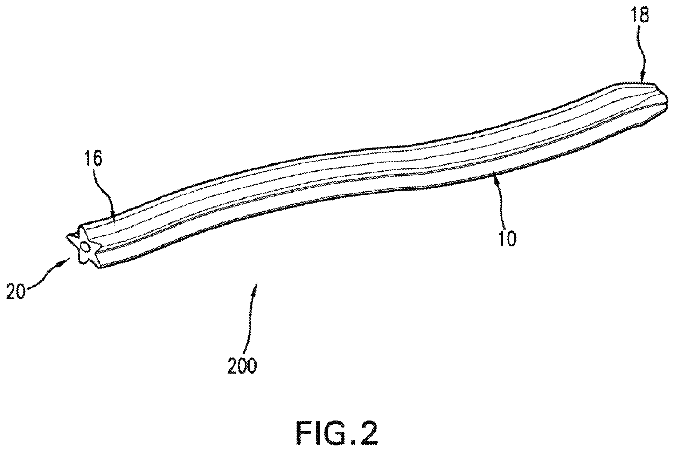

FIG. 2 shows another stent disclosed herein. In this instance, stent 200 has a modified sinusoidial body shape to improve flexibility, allow for varying flow dynamics, and facilitate contour and wall adherence to the lumens. The multiple V shaped channels 16 allow for the flow of various body fluids. The diameter of the internal lumen 20 and the outer diameter of the stent body can be changed based on the need for various luminal dimensions, shapes, flows, and biomechanics. The tapered tip 18 facilitates advancement of the stent inside a body lumen.

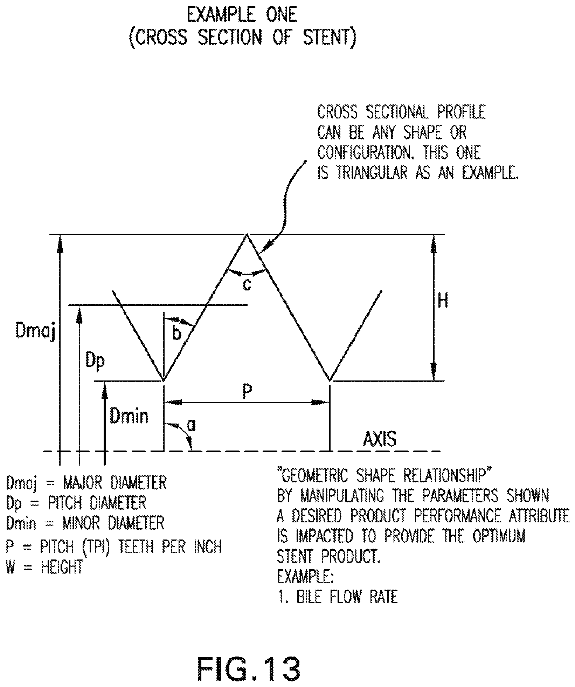

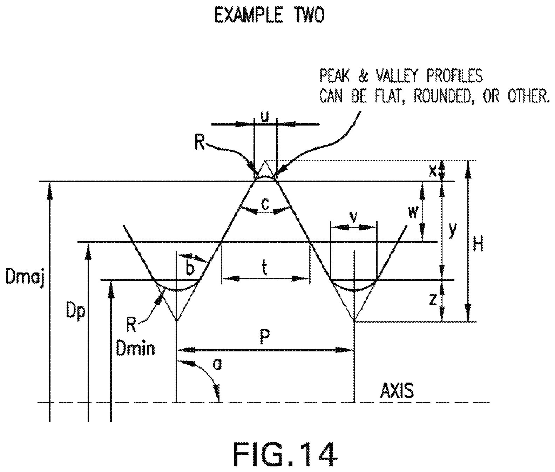

FIGS. 13 and 14 show cross-sections of V-shaped channel and channel walls. The channels can be of varying depths and varying widths to change the volume and speed of fluid flow. The bottom of the channel can be rounded or tapered or formed by a direct angle.

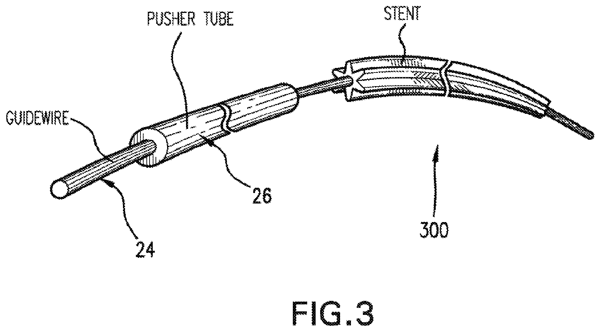

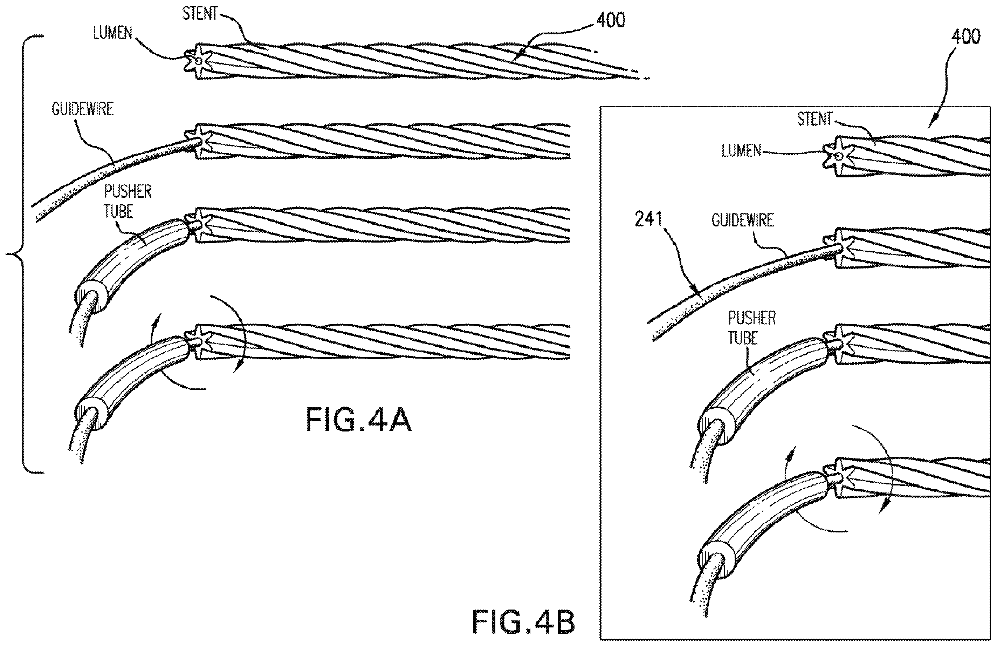

The stent of the present application may be implanted with procedures well known to a person of ordinary skill in the art. Examples of such procedures include, but are not limited to, standard percutaneous approach using a guide wire, endoscopic retrograde cholangiopancreatography (ERCP) placement procedures, and other radiographic/angiographic procedures. FIG. 3 shows an assembly of a stent 200 with a guide wire 24 and a pusher tube 26. FIG. 4 shows several engagement mechanisms among the stent 300, the guide wire 24 and the pusher 26. In FIG. 4A, the pusher tube has several fingers to hold the stent 300 like a hand or clamp. In FIG. 4B, the pusher 26 interlocks with the stent 300 in a male/female fashion to ensure security of positioning and delivery of the stent 300. The interlocking mechanism may involve a male to female interconnect of various shapes, sizes, or dimensions.

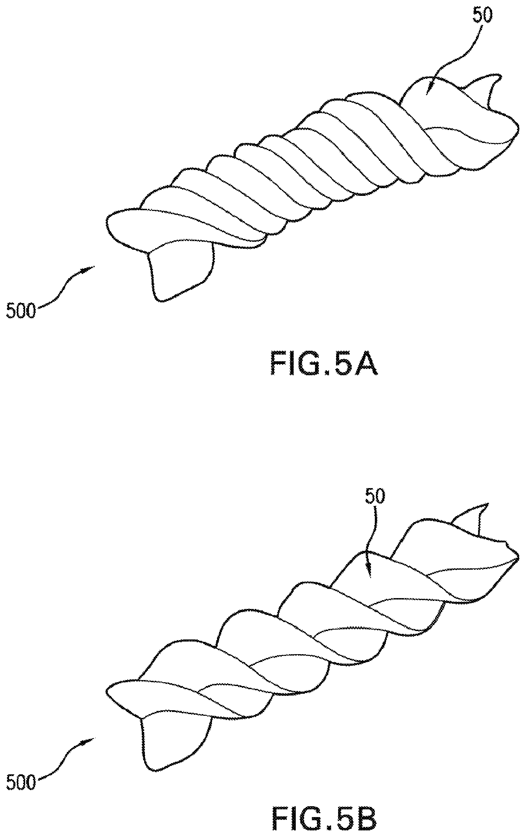

FIGS. 5 A and 5B show an expandable stent 500 disclosed herein with compressible channel walls 50. In a closed state (FIG. 5A), the channel walls 50 are compressed or twisted against each other to reduce stent diameter. Once the stent has been transported to the treatment site, the channel walls are restored to their natural shape (FIG. 5B).

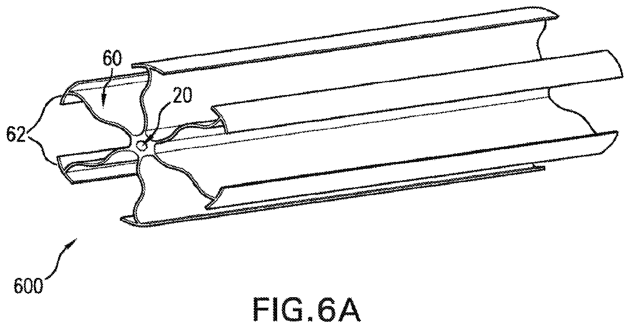

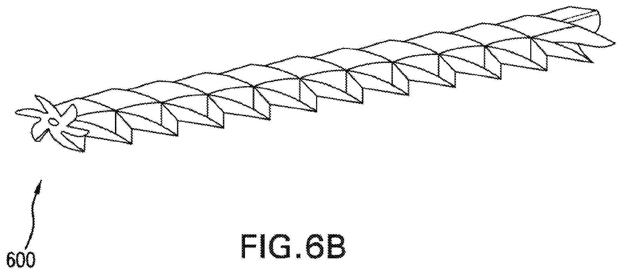

FIGS. 6A and 6B show another expandable stent 600 disclosed herein with foldable leaflets 60. In an extended state, the thin leaflets 60 allow for unobstructed flow of body fluid (FIG. 6A). The leaflets 60 are contoured and aligned in a way to increase the flow speed of the body fluid or to provide minimal drag. The impedance of the flow volumes and the velocity can be modulated by changing the angles and contour of the leaflets. Additionally the interconnecting supports can be thicker at the cam to provide different levels of stability and rigidity for the bracing arms 62, which help support the structure they are placed in. The bracing arms 62 can be connected at anywhere along their diameter and the change in connection points will have an impact in the rigidity of the support of the lumen, the ability of the device to flex with the normal body movement of the lumen, and will change the minimal diameter the device can be collapsed in. The stent 600 may further contain a center lumen 20.

As shown in FIG. 6B, the leaflets 60 may be rotated pivotally (e.g., clockwise) to collapse into each other to reduce the size of the stent to facilitate implantation. Once in place, the stent may be rotated in an opposite direction (e.g., counter clockwise) to restore to its extended state. The tip of the stent 600 can be titled or coned or shaped into various configurations to allow for access to different body lumens. The opened leaflets 60 further have the benefit to prevent migration of the stent 600.

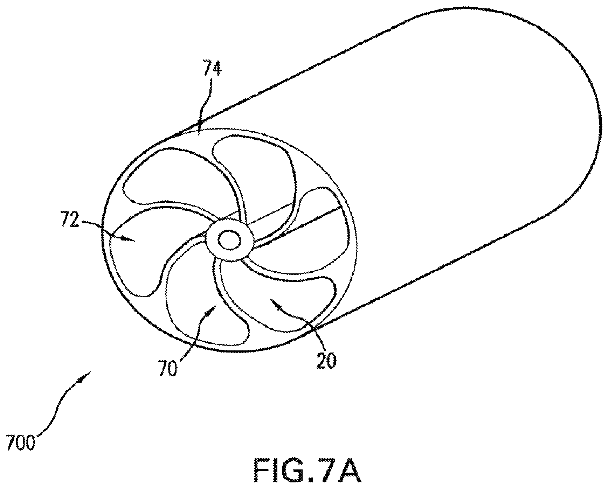

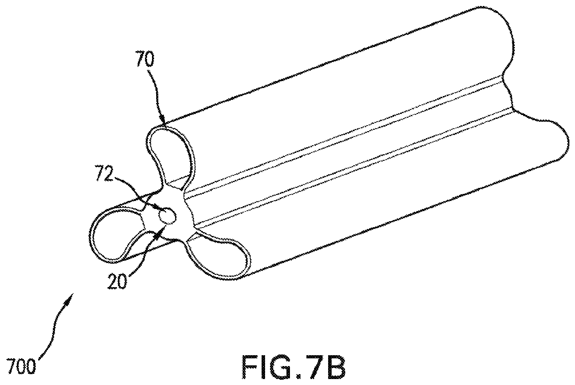

FIGS. 7 A and 7B show another stent. In this instance, the expandable stent 700 disclosed herein has closed connections around each alternating leaflet 70 to allow for changes in flexibility, radial force, compression resistance, and absorption. The leaflets 70 have a sinusoidal pattern and can be thicker at the attachment to inner cam 72 to allow for variations of rigidity. The outer cam 74 prevents tissue growth inside the stent body and increases contact area between the stent 700 and inner wall of the body lumen. The thickness of outer cam 74 is application dependent. The outer cam 74 may also be beveled. The stent 700 may have a removal grip attached to the end of center lumen 20 to allow for easy removal of the stent 700.

FIG. 7B shows another instance of the stent 700. In this instance, the leaflets 70 are connected to the cam 72 and can be compress down towards the cam 74. The leaflets may have a hollow interior so that fluid may flow through and around the leaflets 70.

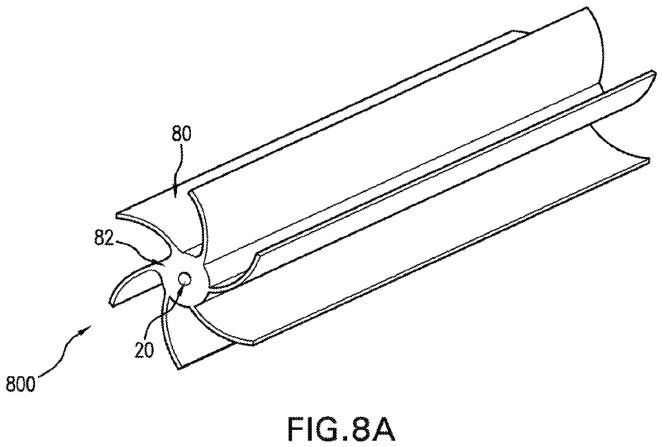

In another instance, stent 800 disclosed herein contains propeller-like leaflets 80 that are thicker at the base where they are attached to the cam or rod portion 82 of the stent 800. The leaflets 80 become thinner at the tip (FIG. 8A). The stent 800 may also have a sinusoidal shape to conform to a body lumen.

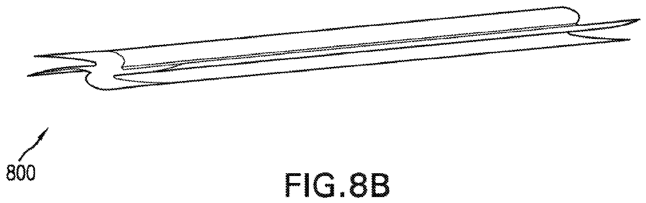

The propeller-like stent 800 may be constructed in such a way to allow unidirectional collapse of the leaflets to facilitate ease of passage through the working channel of an endoscope, bronchoscope, or through some other tubular delivery apparatus or opening by simply rotating the stent in a unidirectional manner and then reversing the technique to open the stent once it is in place. Additionally, the tip of the stent 800 may be shaped to allow for ease of collapse or insertion. FIG. 8B shows a stent 800 in a collapsed configuration.

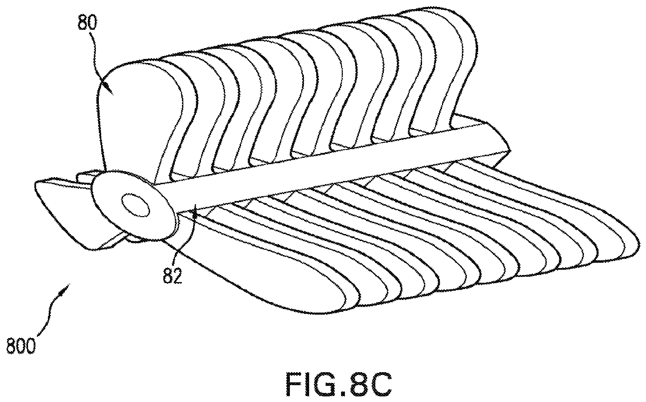

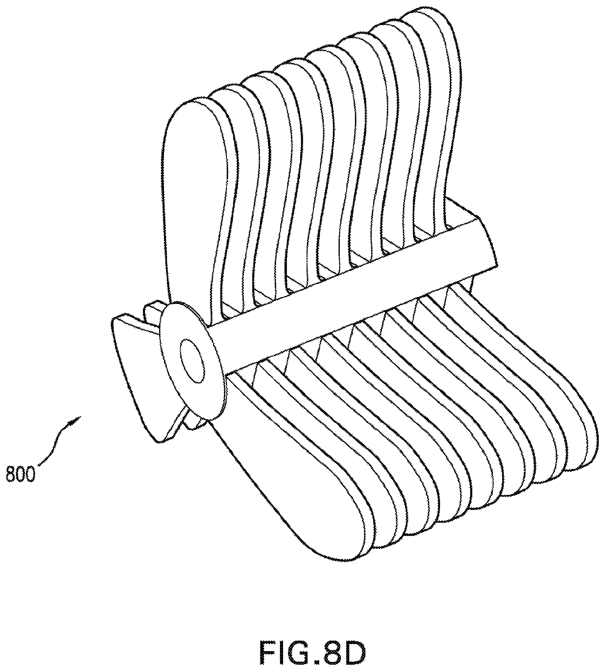

FIG. 8C shows another instance of the stent 800. In this instance, the leaflets 80 can be folded towards the cam or rod portion 82 of the stent body, in a manner similar to that of an umbrella. The leaflets 80 can be in any shape, such as round, oval, triangle etc. and will have a change in thickness at the base where the leaflets are connected to the cam 82 to allow you to change ease or rigidity of folding the device and passing it through and opening or channel. The unidirectional leaflets allow the device to be pushed through an opening and then pulled back to secure it in place. In another instance, the leaflets can be folded towards the cam or rod portion of the stent body, in a manner similar to that of an umbrella, a collapsing tree, or unidirectional or multidirectional folding leaflets of consistent or varying shapes. FIG. 8D shows the stent of FIG. 8C in a collapsed configuration.

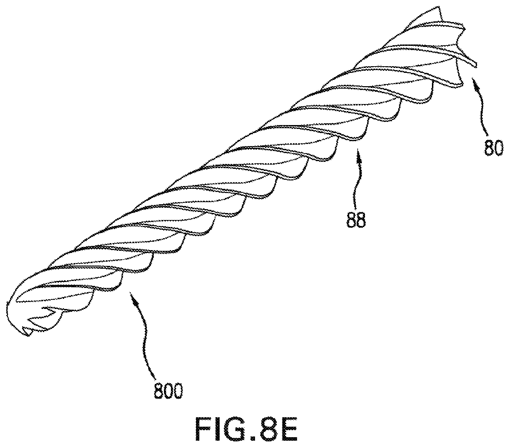

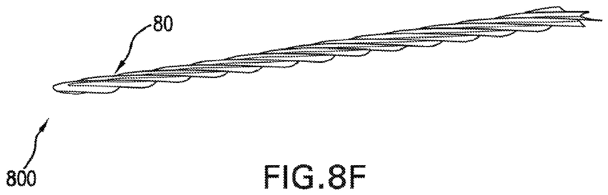

In another instance, the leaflets 80 of the stent 800 can be folded together by rotating along a common axis. FIGS. 8E and 8F show a stent 800 in open and folded configurations, respectively. In one instance, the stent 800 has a diameter of 1 cm in open configuration and a diameter of 1 mm in folded configuration. Depending on flow requirements, the channel 88 may have raps ranging from 5 to 100 degrees. In certain instances, the channel 88 may have raps of about 5-20 degrees, 20-40 degrees, 40-60 degrees, 60-80 degrees or 80-100 degrees. In certain instances, the channel has a rap of about 5 degrees, about 10 degrees, about 20 degrees, about 30 degrees, about 40 degrees, about 50 degrees, about 60 degrees, about 70 degrees, about 80 degrees, about 90 degrees, or about 100 degrees.

In another instance, a device has a portion of the device and stent and its leaflets collapsible so that some portion of the device (e.g., 1%) would have uni-direction leaflets and the remainder would have the opposite facing leaflets or directions such as seen on the different blades of a saw. In yet another instance, the leaflets are alternating in directions so as to prevent migration of the expanded stent.

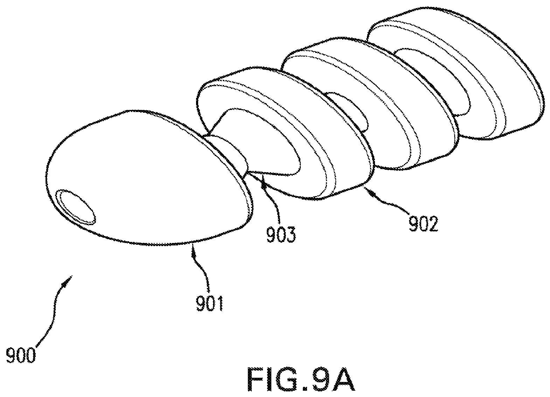

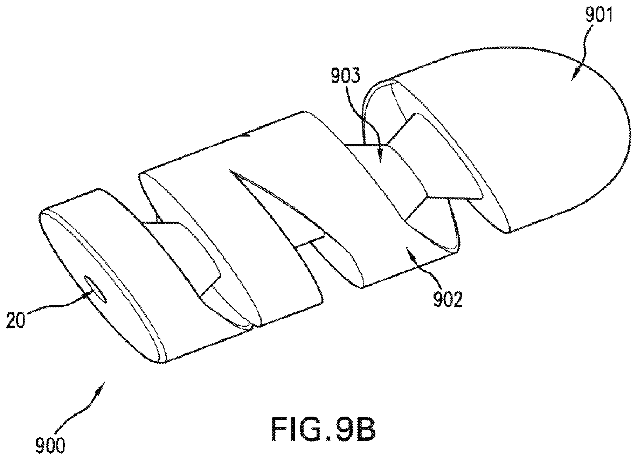

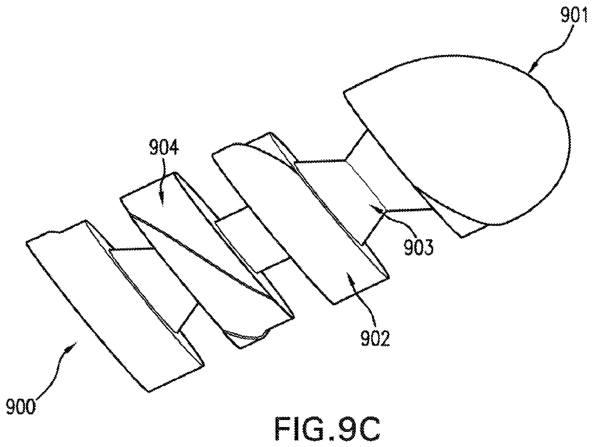

Referring now to FIG. 9A an instance of stent 900 disclosed herein has a tapered proximal end 901 to allow ease of passage inside a body lumen, an outer frame 902 with a larger diameter to provide stiffness, and a center core 903 with a smaller diameter to provide flexibility. The outer frame 902 and the center core 903 can be cylindrical or cut with various contours in the surface to change the flexibility or rigidity of the stent. In FIG. 9B, the stent 900 has an outer frame 902 that forms a coil around the core 903. The stent 900 disclosed herein may further include a center lumen 20. In FIG. 9C, the stent 900 has sinusoidal channel 904 formed on the surface of outer frame 902. The channel 904 may have variable depth. The center core 903 may have various shapes and sizes to adjust the flexibility, stability and rigidity of the stent 900.

In one instance, the stent 900 is inserted into the canal of a bone having a fracture. In another instance, the stent 900 is coated with a hydrogel. The hydrogel expands by absorbing of fluids and improves the connection and support of the inner wall of the bone canal. In another instance, the stent 900 is used to attach bone fractures together. In another instance, the stent 900 is placed through the bone cortex.

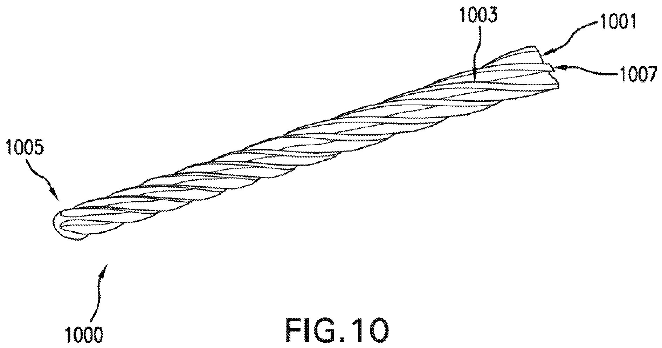

Referring now to FIG. 10, another instance of a stent 1000 disclosed herein has channels of varying widths and depths on the exterior of the stent body. For example channel 1001 has a width that is greater than the width of channel 1003. The variable width and depth can be used to change the flow of fluids or friction to the lumen it is place in. Similar channels may also be formed on the interior side of a tubular stent. In the instance shown in FIG. 10, the stent 1000 has a tapered tip 1005 to facilitate advancement of the stent inside a body lumen. The wide distal flare 1007 prevents migration and increases stability of the stent 900. In one instance, the distal flare 1007 extends past the body of the stent 100 into a tail or a flap. The stent 1000 may have channels of shorter or longer pitches to enable increases in fluid flow and stability. The stent 1000 may further include a center lumen for a guide wire or fluid flow.

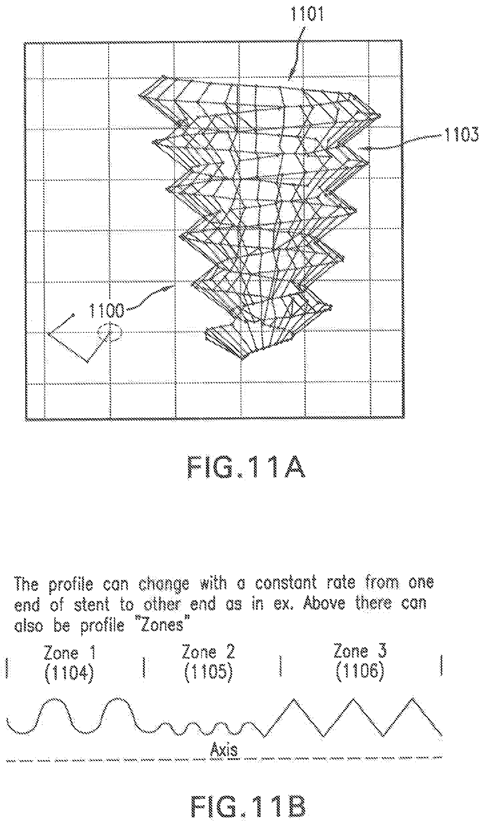

Referring now to FIGS. 11A-11B another instance of a stent 1100 disclosed herein has a larger proximal end 1101 with a helical surface channel 1103. The stent 1100 is in the shape of a cone or a cylinder with alternating variation in the diameter of the stent body. The surface channel 1103 may have regions 1104, 1105 and 1106 with different shapes and depth in each region, so as to change the flow rate, flow volume, and/or in each region.

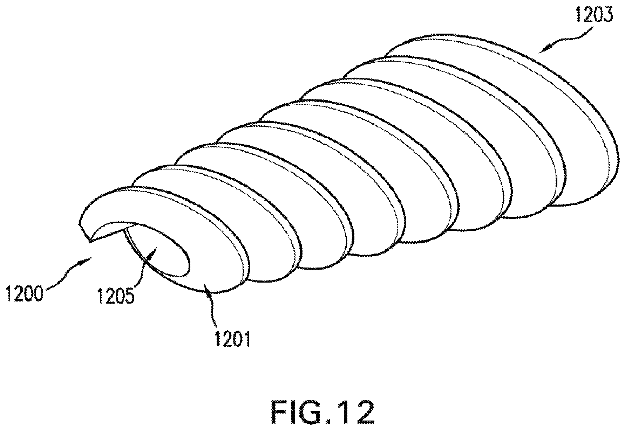

FIG. 12 shows another instance of a stent 1200 disclosed herein. The pitch of the stent can change in various zones of the stent. The stent has a smaller diameter in the proximal end 1201 and larger diameter in the distal end 1203. The stent 1200 may have an opening 1205 that is big enough to adapt a wire. The stent 1200 may have a gradual increasing or decreasing pitch. In another instance, the pitch may change in different sections of the stent to better contour to the anatomy.

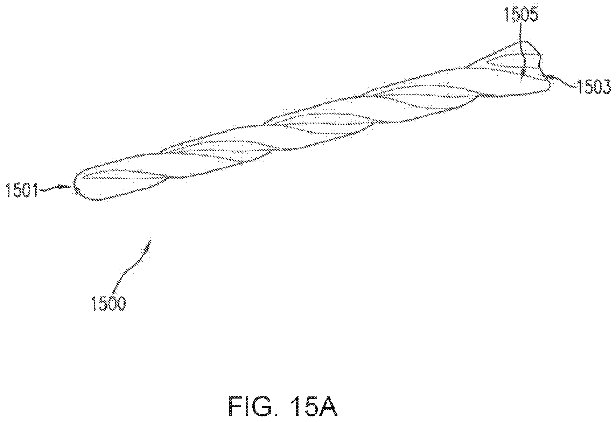

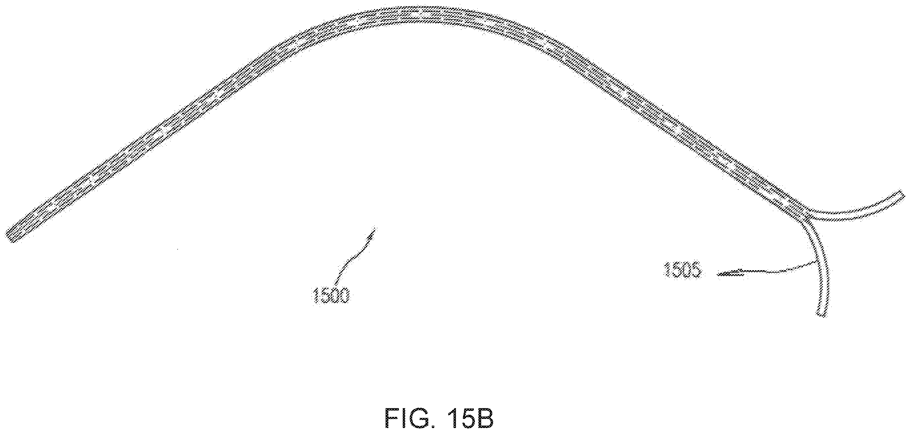

Other stents of the present disclosure are shown in FIGS. 15-18.

FIG. 15A shows a stent 1500 with a conical tip 1501 to allow for ease of access into the area it will be placed, a flared distal end 1503 for anchoring or prevention of migration into a lumen, out of a lumen, or within a lumen. The flares 1505 can be unidirectional or bi directional.

In the invention, the flares 1505 are extended past the body of the stent 1500, as shown in FIG. 15B, thereby forming flaps or tails. The flares 1505 are Y-shaped with respect to the body of the stent 1500.. The stent 1500 may include the flares 1505 in a form of flaps or tails on one or both ends of the stent 1500 body. In addition, the stent 1500 may include the flares 1505 in a form of flaps or tails in proximity of one or both ends of the stent 1500 body. Additionally, one or more flares 1505 may be flaps arranged anywhere along the body of the stent 1500.

One or more flares 1505 may be flaps arranged symmetrically with respect to the stent 1500 axis, or may be positioned anywhere along the circumference of the stent 1500 body with respect to each other. In one embodiment, the flares 1505 are located at different longitudinal positions along the stent 1500 body, or they may be placed at the same longitudinal location on the stent 1500 body.

The flares 1505 are elongated protrusions that extend from the stent and contact the tissue of the lumen in order to hold the stent in place. In some embodiments, the tips of the flares 1505 are pointed, so that the tips can embed in the tissue. In some embodiments, the anti-migration flares 1505 are held flush with the surface of the stent prior to and during deployment, and are allowed to fold out following placement at the target site. In one instance, the anti-migration flares 1505 are placed in proximity to the ends of the stent, or at the very ends of the stent. However, one of ordinary skill will understand that the placement of the anti-migration flares 1505 is not limiting on the application. In some embodiments, the anti-migration flares 1505 are between about 1 mm and about 12 mm in length beyond the stent 1500 body. In further embodiments, the anti-migration flares 1505 are between about 3 mm and about 10 mm in length beyond the stent 1500 body. In still further embodiments, the anti-migration flares 1505 are between about 5 mm and about 8 mm in length beyond the stent 1500 body. In a particular embodiment, the anti-migration flares 1505 are about 7 mm in length beyond the stent 1500 body.

In some embodiments, the flares 1505 extend in a Y-shape away from the stent 1500 body, as shown in FIG. 15B. One of the flares 1505 may be longer than the other, or they may be the same in length. Moreover, one of the flares 1505 may be thicker than the other, or they may be of the same thickness. In one example, the flares 1505 are cylindrical, and a diameter of one of the flares 1505 is greater than the other. In another example, the flares 1505 are of the same diameter. In one example, the length and the thickness of the flares 1505 are determined based on expected size of the lumen in which the stent 1500 is inserted in and the external diameter of the stent 1500 body.

In one embodiment, the flares 1505 may be rigid, and in another embodiment they may be flexible. The flexibility of one of the flares 1505 may be greater than that of the other one, or they may be of the same thickness. In one example, the flexibility of the flares 1505 are determined based on expected size of the lumen in which the stent 1500 is inserted in and the external diameter of the stent 1500. In another example, the flexibility of the flares 1505 depends on an expected path prior to implantation, and specifically, the curvatures of a trajectory that the stent 1500 needs to travel through prior to the arrival at the target site. In yet another example, the flexibility of the flares 1505 depends on the surrounding lumen tissue around the implanted stent 1500.

In one embodiment, the flares 1505 are tucked-in, in order to remain flush with the body of the stent 1500 prior to the arrival at the target site. In another embodiment, the flares 1505 are wrapped together in a shell and kept aligned with the stent 1500 body in order to facilitate stent transportation to the target site. In one example, the shell is removed prior to deploying the stent 1500 in order to release the flares 1505 and anchor the stent 1500 in place. The shell removal may be performed by pulling the shell off of the flares 1505, or, in the alternative, the shell is biodegradable and it dissolves in order to release the flares 1505. The shell may be mounted over the flares 1505 and removed prior to stent deployment in any other manner deemed suitable.

In one example, the flares 1505 are made of the same material, or, in the alternative, they may be mounted on the stent 1500 body made from different biodegradable materials. The flares 1505 may be made from the same biodegradable material as the stent 1500 or from a different material, for example, with an objective for the anti-migratory flares 1505 to dissolve slower than the stent 1500 body in order to keep the stent 1500 anchored into its accommodating lumen while the stent 1500 biodegrades.

In the invention, the flares 1505 are flat at their ends. The flares 1505 may have radiused ends, or may be of any other shape deemed suitable for the implantation site.. The flares 1505 may be of the same shape or of any combination of different shapes deemed appropriate for implantation and anti-migration purposes.

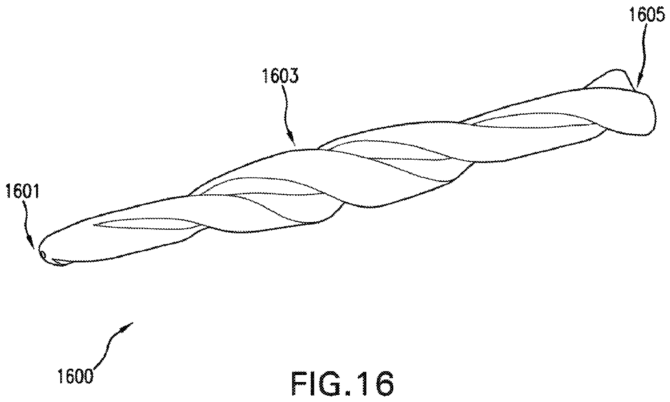

FIG. 16 shows a stent 1600 disclosed herein with a conical end 1601 and a swollen middle section 1603. The stent 1600 may be made from an elastomer. In one instance, the elastomer may expands more in a area in the middle, the end, or in multiple locations of the stent body to increase fluid flow by providing larger and deeper channels in the stent. In another instance, the end of the stent 1600 has an anti-migration mechanism that will expand to keep the stent in place. Anti-migration device at the distal end 1605 of the stent can be located anywhere along the length of the stent access.

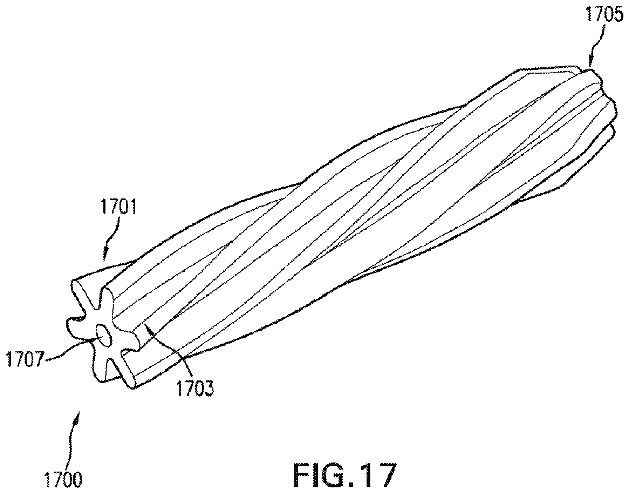

FIG. 17 shows another instance of a stent 1700 disclosed herein with leaflets 1701 to form channels 1703. In this instance, the stent 1700 has a tapered end 1705 to allow for ease of entry. The rotation of the sinusoidal channels 1703 may be changed to adjust fluid flow, collapse ability, etc. The leaflets attached to the cam 1707 can be folded over to allow the diameter of the stent 1700 to become smaller when being loaded into a deliver device or being place in a deliver tube like an endoscope. The channels walls can be straight, rounded, or a combination thereof depending on the cavity or lumen where the stent is placed.

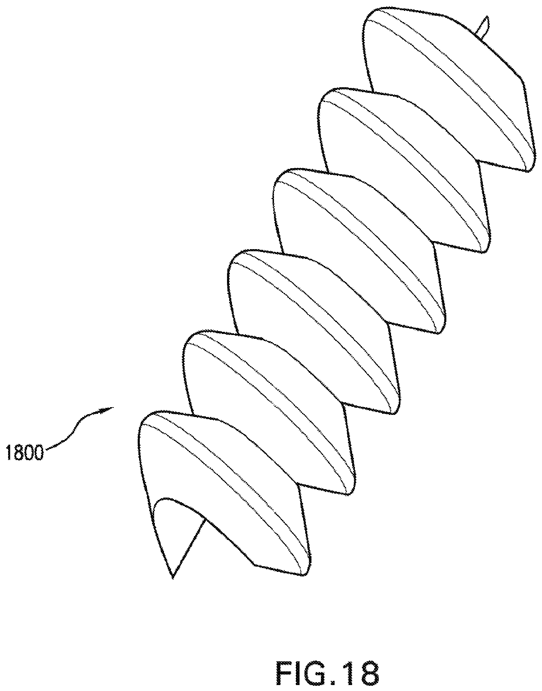

FIG. 18 shows another stent 1800. The stent 1800 is made in a way to allow the sinusoidal channel of the stent occur on the inside of the stent. The outside of the stent conforms to the anatomy the stent is placed in and flexibility is determined by the pitch of the sinusoidal channel. The inside of the stent forms the same sinusoidal as the outside of the stent. In one instance, the stent 1800 is made in such a way that it can be inserted in a screw in fashion.

A person or ordinary skill in the art would understand that other folding or interlocking may also be employed. The channel walls or leaflets can also be of varying thicknesses and lengths to provide the stent with desired rigidity, flexibility, pushability, trackability, luminal contact and/or absorption profile. For example, a stent made from bioabsorbable material may have leaflets that are thinner at the tip (where they touch the lumen wall) and thicker at the base (where they are attached to the cam), thus allowing for degradation from the tip to the base. In another instance, the cam itself can be cut in various ways to change its diameter at different points to change the pushability and flexibility of the device.

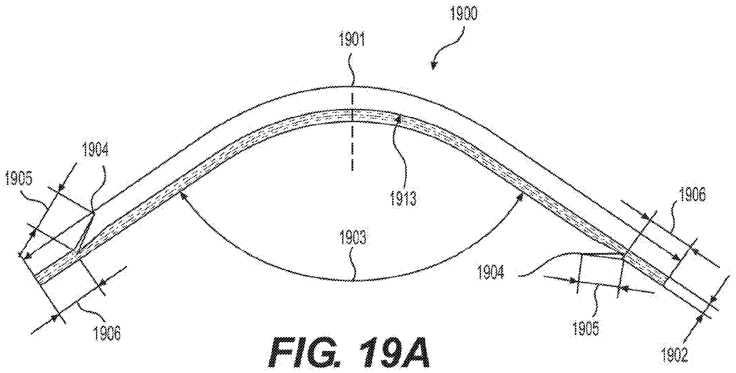

FIG. 19A shows the basic design of another embodiment of a stent 1900. In one embodiment, the stent 1900 is made from a polymeric material. In certain embodiments, the polymeric material may be Aquaprene 8020 with Opaciprene, Dioxaprene 100M with Opaciprene, or Lactoprene 7415 with Opaciprene. Appropriate grades of USD, PPD and MDP may also be selected for use in manufacturing the stent. In some embodiments, the stent comprises a fast-absorbing polymer, such as USD5 (Aquaprene 8020: 20% PEG, 80% p-dioxanone). In other embodiments, the stent comprises a medium-absorbing polymer, such as PPD3 (Dioxaprene 100M: Poly(para-diaxanone). In still other embodiments, the stent comprises a slow-absorbing polymer, such as MDP3 (Lactoprene 7415: 74/15/11 copolymer of lactide/trimethylene carbonate/caprolactone). In some embodiments, the lactide is the monomer L-lactide. In some embodiments, the stent is impregnated with a ~1% to -40% BaS04 solution in a suitable carrier. In further embodiments, the stent is impregnated with a -10% to -30% BaS04 solution in a suitable carrier. In still further embodiments, the stent is impregnated with a -12% to -22% BaSCri solution in a suitable carrier. In particular embodiments, the stent is impregnated with a -17% BaSCri solution in a suitable carrier.

The stent length 1901 is variable, dependent upon the application or location the stent 1900 is to be used in. In some embodiments, the stent length 1091 can be between about 5 mm and about 300 mm. In particular embodiments, the stent length 1091 is about 20, 40, 60, 80, 100, 150 or 225 mm. In some embodiments, the stent 1900 has an outer diameter 1902 of between about 1.8 mm and about 2.2 mm. In other embodiments, the stent 1900 has an outer diameter 1902 of between about 1.9 mm and about 2.1 mm. In particular embodiments, the stent 1900 has an outer diameter 1902 of about 2.0 mm. In some embodiments, the ends of the stent 1900 are tapered to be narrower than the main body of the stent 1900. The "outer diameter" refers to the linear distance between the two farthest points on the device along a straight line that passes through the center of the device in a cross-section.

In some embodiments, the biliary and pancreatic stents of the present application are made for materials that are visible fluoroscopically such that the stent can be monitored under fluoroscopy. In comparison to other stents, the biliary and pancreatic stents of the present application provide better simulated flow rates, better simulated migration resistance and better crush resistance.

The stent 1900 of this embodiment is flexible. In some embodiments, the stent flexes after placement in the target location. In some embodiments, the body of the stent can tolerate a bend 1903 of between about 90° and about 135° without experiencing a degradation of fluid flow rate. In other embodiments, the body of the stent can tolerate a bend 1903 of between about 100° and about 125° without experiencing a degradation of fluid flow rate. In still other embodiments, the body of the stent can tolerate a bend 1903 of about 112° without experiencing a degradation of fluid flow rate.

In some embodiments, the body of the stent 1900 is curved, having a curvature 1913 with a radius of between about 10 mm and about 70 mm. In other embodiments, the body of the stent 1900 has a curvature 1913 with a radius of between about 20 mm and about 60 mm. In still other embodiments, the body of the stent 1900 has a curvature 1913 with a radius of between about 30 mm and about 50 mm. In particular embodiments, the body of the stent 1900 has a curvature 1913 with a radius of about 40 mm.

The stent 1900 comprises two anti-migration devices 1904 that expand outwards from the elongated body of the stent 1900 so as to anchor the stent in position within a bodily lumen. In some embodiments, the anti-migration devices are elongated protrusions that extend from the stent and contact the tissue of the lumen in order to hold the stent in place. It some embodiments, the tip of the anti-migration devices is pointed, so that the tip can embed in the tissue. In some embodiments, the anti-migration devices are held flush with the surface of the stent prior to and during deployment and are allowed to fold out following placement at the target site. The anti-migration devices 1904 are placed in proximity to the ends of the stent, however, one of ordinary skill will understand that the placement of the anti-migration devices 1904 is not limiting on the application. In some embodiments, the anti-migration devices 1904 are between about 1 mm and about 12 mm in length 1905. In further embodiments, the anti-migration devices 1904 are between about 3 mm and about 10 mm in length 1905. In still further embodiments, the anti-migration devices 1904 are between about 5 mm and about 8 mm in length 1905. In a particular embodiment, the anti-migration devices 1904 are about 7 mm in length 1905. Furthermore, in some embodiments, the anti-migration devices 1904 are a distance 1906 of between about 1 mm and about 12 mm from each end of the stent 1900. In further embodiments, the anti-migration devices 1904 are a distance 1906 of between about 3 mm and about 10 mm from each end of the stent 1900. In still further embodiments, the anti-migration devices 1904 are a distance 1906 of between about 5 mm and about 8 mm from each end of the stent 1900. In a particular embodiment, the anti-migration devices 1904 are a distance 1906 of about 7 mm from each end of the stent 1900. In some embodiments, the anti-migration devices 1904 are the same distance 1906 from each end of the stent 1900. In other embodiments, the anti-migration devices 1904 are different distances 1906 from each end of the stent 1900. In some embodiments, the anti-migration devices 1904 are in the form of flaps or tails 1910.

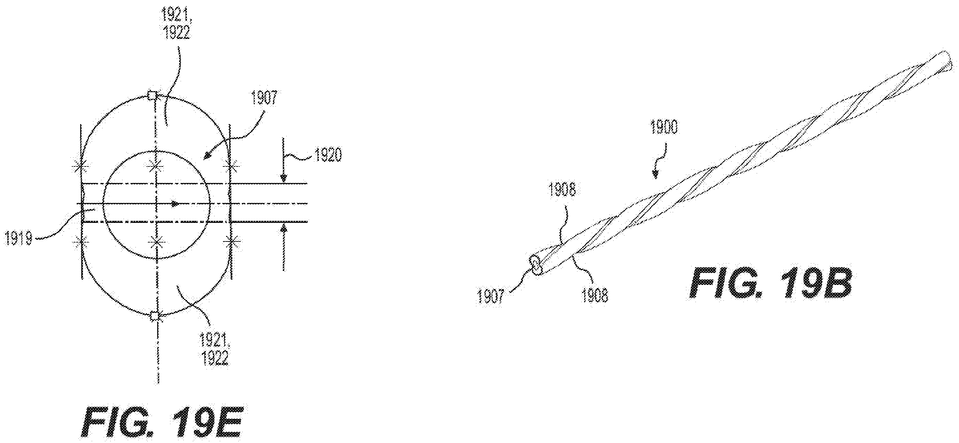

FIG. 19B is a 3-D rendering of the stent 1900. The end of this perspective view shows that this embodiment has a single central lumen 1907. The longitudinal central lumen 1907 of the stent 1900 is formed within the polymeric material and provides a channel for a guide wire. The narrowing of the stent on either side of the central lumen 1907 creates opposing external channels 1908 for fluid flow on the external surface of the stent 1900. As shown in FIG. 19B, the stent 1900 is twisted, causing the opposing external channels 1908 to spiral around the stent 1900.

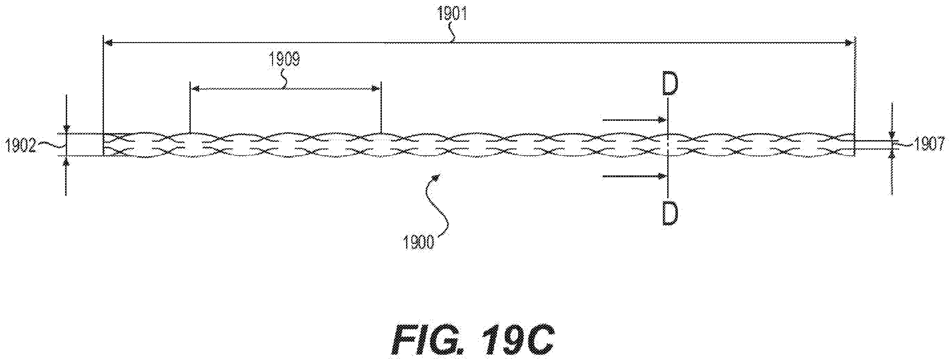

FIG. 19C is a side view of the stent 1900, showing the spiraling of the external channels around the central lumen 1907 (dashed lines). The present inventors have surprisingly found that the fluid flow through the external channels is optimized by controlling the number of twists of the stent per inch (i.e., number of turns per 25.4 mm, shown in FIG. 19C as 1909, wherein one twist is a 360 degree rotation around the central axis of the stent). In addition, the present inventors have found that increasing the number of twists per inch (TPI) increases/improves the flexibility of the stent, as measured by deflection of the stent material with an equivalent amount of force applied. Increasing the TPI (for example, from 1 TPI to 2 TPI or from 2 TPI to 3 TPI) increases the amount of deflection, thereby indicating that higher TPI produces a more flexible stent. In some embodiments, the number of twists is at least, or more than, 1 twist per inch. In other embodiments, the number of twists is at least, or more than, 1.5 twists per inch. In still other embodiments, the number of twists is at least, or more than, 1.75 twists per inch. In yet other embodiments, the number of twists is between about 1.5 and 2.5 twists per inch. In even other embodiments, the number of twists is between about 1.75 and 2.25 twists per inch. In still other embodiments, the number of twists is about 2 twists per inch. In particular embodiments, the number of twists is at least, or more than, 2 twists per inch. In further embodiments, the number of twists equals 2 twists per inch. In other further embodiments, the number of twists is more than 2 twists per inch. The twisting of the stent may be carried out by any means known to one of ordinary skill in the art. In some embodiments, the stent may be formed in a straight, non-twisted, configuration, followed by twisting or machining to the desired number of twists/inch. In other embodiments, the stent 1900 may be formed or molded in the twisted shape. The polymer material from which the stent 1900 is made may be fixed in place in the twisted position by any means known to one of ordinary skill in the art, e.g., a heating process, etc.

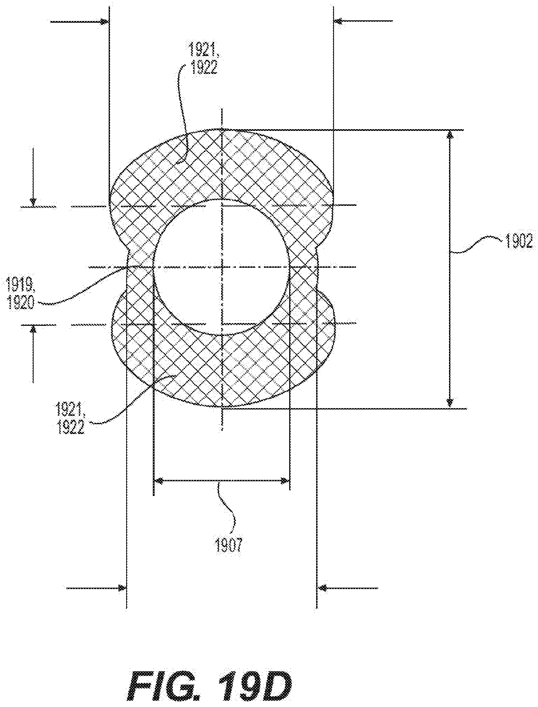

FIG. 19D shows a cross-section view of the stent 1900 at line D-D of FIG. 19C and looking towards one end of the stent 1900. In terms of orientation for describing the cross-section, the directions up, down, upper and lower refer direction lying on the main axis that bisects the cross-section of the stent into the longest mirror-image halves. Sides and side-to-side refer to a cross axis that is perpendicular to the main axis. In some embodiments, the central channel 1907 is between about 0.8 mm and about 1.2 mm in diameter. In other embodiments, the central channel 1907 is between about 0.9 mm and about 1.1 mm in diameter. In still other embodiments, the central channel 1907 is between about 0.95 mm and about 1.05 mm in diameter. In particular embodiments, the central channel 1907 is about 1 mm in diameter. In other particular embodiments, the central channel 1907 is 1 mm +/- 0.1 mm in diameter. The "diameter" of the central channel refers to the linear distance between the two farthest points within the lumen of the central channel along a straight line that passes through the center of the central channel in a cross-section.

Still referring to FIG. 19D, the cross-section of the stent comprises a central circle 1919 that surrounds the central channel 1907 and upper and lower bolsters 1921 that overlap the central circle 1919 and, in some embodiments, each other. The sides of the central circle 1919, between the points where the bolsters 1921 intersect with the central circle 1919, form the minor, thinner, walls 1920 of the stent around the central channel 1907. In some embodiments, the thickness of the minor walls 1920 is between about 0.1 mm and about 0.3 mm. In other embodiments, the thickness of the minor walls 1920 is between about 0.15 mm and about 0.25 mm. In particular embodiments, the thickness of the minor walls 1920 is about 0.2 mm +/- 0.02 mm. In more particular embodiments, the thickness of the minor walls 1920 is about 0.2 mm +/- 0.01 mm.

Still referring to FIG. 19D, the upper and lower bolsters 1921 are generally elliptical, oval or circular in shape. In the case of an oval or elliptical bolster 1921, the longest longitudinal axis of the bolster 1921 may he oriented perpendicular to the main axis of the cross section of the stent, as shown in FIG. 19D. In some embodiments, the longest longitudinal axis of an oval or elliptical bolster 1921 may he oriented along the main axis of the cross section of the stent. The bolsters 1921 form the major, thicker, walls of the stent.

In some embodiments, the thickness of the major walls 1922 is between about 0.3 mm and about 0.7 mm at the main axis. In other embodiments, the thickness of the major walls 1922 is between about 0.4 mm and about 0.6 mm at the main axis. In particular embodiments, the thickness of the major walls 1922 is about 0.5 mm +/- 0.05 mm at the main axis. In more particular embodiments, the thickness of the major walls 1922 is about 0.5 mm +/- 0.025 mm at the main axis.

In some embodiments, the bolsters 1921 have a side-to-side thickness of between about 1.4 mm and about 1.8 mm. In other embodiments, the bolsters 1921 have a side-to-side thickness of between about 1.5 mm and about 1.78 mm. In particular embodiments, the bolsters 1921 have a side-to-side thickness of about 1.61 mm +/- 0.16 mm. In more particular embodiments, the bolsters 1921 have a side-to-side thickness of about 1.61 mm +/- 0.08 mm.

FIG. 19E shows a cross-sectional view of an alternative embodiment of the stent 1900 at line D-D of FIG. 19C and looking towards one end of the stent 1900 where the bolsters 1921 are of a circular shape. The sides of the central circle 1919, between the points where the bolsters 1921 intersect with the central circle 1919, form the minor, thinner, walls 1920 of the stent around the central channel 1907. In some embodiments, the thickness of the minor walls 1920 is between about 0.1 mm and about 0.3 mm. In other embodiments, the thickness of the minor walls 1920 is between about 0.15 mm and about 0.25 mm. In particular embodiments, the thickness of the minor walls 1920 is about 0.2 mm +/- 0.02 mm. In more particular embodiments, the thickness of the minor walls 1920 is about 0.2 mm +/- 0.01 mm. In some embodiments, the diameter of the circular bolsters 1921 is the same as the diameter of the central circle 1919. In other embodiments, the diameter of the circular bolsters 1921 is greater than the diameter of the central circle 1919. In some embodiments, the thickness of the major walls 1922 is between about 0.3 mm and about 0.7 mm at the main axis. In other embodiments, the thickness of the major walls 1922 is between about 0.4 mm and about 0.6 mm at the main axis. In particular embodiments, the thickness of the major walls 1922 is about 0.5 mm +/-0.05 mm at the main axis. In more particular embodiments, the thickness of the major walls 1922 is about 0.5 mm +/- 0.025 mm at the main axis.

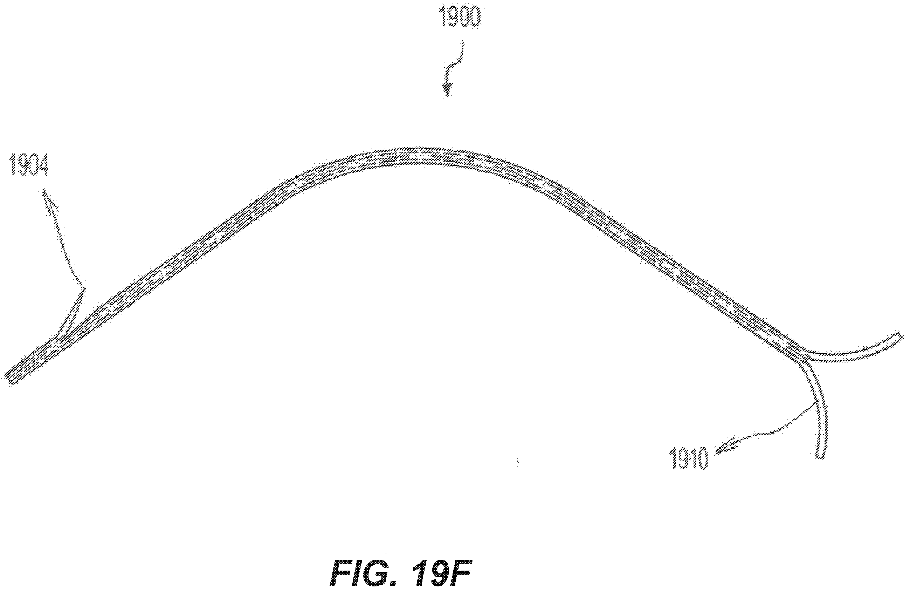

FIG. 19F shows a stent 1900 comprising an anti-migration device 1904 and two anti-migration tails 1910. In one example, the anti-migration device 1904 expands outwards from the elongated body of the stent 1900 so as to anchor the stent 1900 in position within a bodily lumen. The anti-migration tails 1910 extend beyond the elongated body of the stent 1900 also to anchor the stent in position within a bodily lumen. In the present invention, the anti-migration tails 1910 form a Y-shape with the body of the stent 1900.

The anti-migration tails 1910 are elongated protrusions that extend from the stent and contact the tissue of the lumen in order to hold the stent in place. It some embodiments, the tips of the anti-migration tails 1910 are pointed, so that the tips can embed in the tissue. In some embodiments, the anti-migration tails 1910 are held flush with the surface of the stent prior to and during deployment, and are allowed to fold out following placement at the target site. The anti-migration tails 1910 may be placed in proximity to the ends of the stent, or at the very ends of the stent. In some embodiments, the anti-migration tails 1910 are between about 1 mm and about 12 mm in length beyond the stent body. In further embodiments, the anti-migration tails 1910 are between about 3 mm and about 10 mm in length beyond the stent body. In still further embodiments, the anti-migration tails 1910 are between about 5 mm and about 8 mm in length beyond the stent body. In a particular embodiment, the anti-migration tails 1910 are about 7 mm in length beyond the stent body.

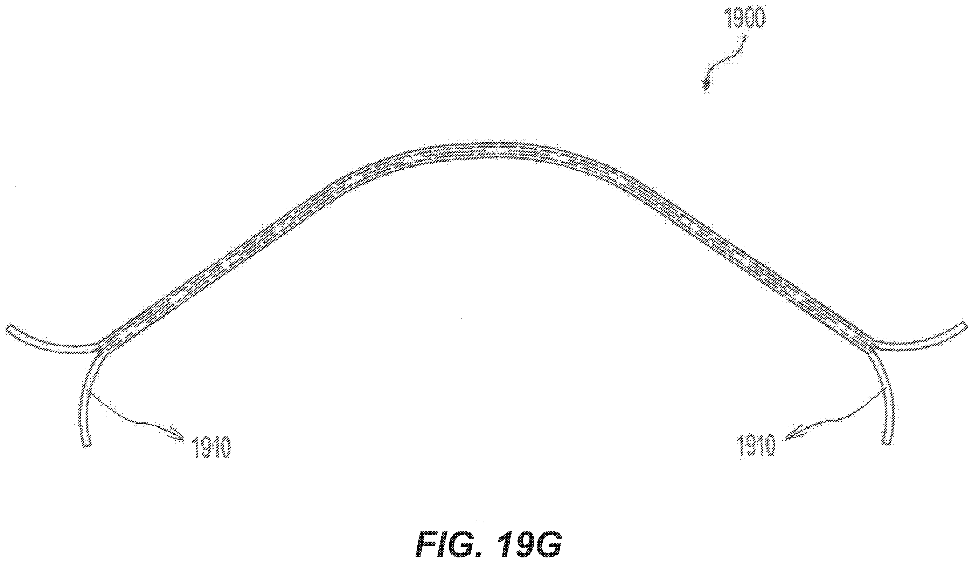

The bi directional tails 1910 of the claimed invention are extended past the body of the stent 1900, as shown in FIGS. 19F and 19G, thereby forming end flaps. In one example, the tails 1910 are two unidirectional extensions. The stent 1900 may include the tails 1910 in a form of flaps or tails on one end of the stent 1900 body, as illustrated in FIG. 19F, or on both ends of the stent 1900 body, as shown in FIG. 19G, for example. In addition, the stent 1900 may include the tails 1910 in a form of flaps or tails in proximity of one or both ends of the stent 1900 body.

Two or more tails 1910 may be flaps arranged symmetrically with respect to the stent 1900 axis, or may be positioned anywhere along the circumference of the stent 1900 body with respect to each other. In one example, the tails 1910 are located at different longitudinal positions along the stent 1900 body, or they may be placed at the same longitudinal location on the stent 1900 body.

The tails 1910 of the present invention extend in a Y-shape away from the stent 1900 body, as shown in FIGS. 19F and 19G. One of the tails 1910 may be longer than the other, or they may be the same in length. Moreover, one of the tails 1910 may be thicker than the other, or they may be of the same thickness. In one embodiment, the tails 1910 are cylindrical in shape, and one of the tails 1910 may be greater in diameter than the other. In another example, the tails 1910 are of the same diameters. In one example, the length and the thickness of the tails 1910 are determined based on expected size of the lumen in which the stent 1900 is inserted in and the external diameter of the stent 1900 body.

In one embodiment, the tails 1910 may be rigid, and in another embodiment they may be flexible. The flexibility of one of the tails 1910 may be greater than that of the other one, or they may be of the same flexibility. In one example, the flexibility of the tails 1910 are determined based on expected size of the lumen in which the stent 1900 is inserted in and the external diameter of the stent 1900. In another example, the flexibility of the tails 1910 depends on an expected path prior to implantation, and specifically, the curvatures of a trajectory that the stent 1900 needs to travel through prior to the arrival at the target site. In yet another example, the flexibility of the tails 1910 depends on the surrounding lumen tissue around the implanted stent 1900.

In one embodiment, the tails 1910 are tucked-in, in order to remain flush with the body of the stent 1900 prior to the arrival at the target site. In another embodiment, the tails 1910 are wrapped together in a shell and kept aligned with the stent 1900 body in order to facilitate their transportation to the target site. The shell may be removed prior to deploying the stent 1900 in order to release the tails 1910 and anchor the stent 1900 in place. The shell removal may be performed by pulling the shell off of the tails 1910, or, in the alternative, the shell may be biogradable and may dissolve in order to enable the release of the tails 1910. The shell may be mounted over the tails 1910 and removed prior to stent deployment in any other manner deemed suitable.

In some embodiments, the tails 1910 are made of the same material, or, in the alternative, they may be mounted on the stent 1900 body made from different biodegradable materials. The tails 1910 may be made from the same biodegradable material as the stent 1900 or from a different material, for example, with an objective for the anti-migratory tails 1910 to dissolve slower than the stent 1900 body in order to keep the stent 1900 anchored into its accommodating lumen while the stent 1900 biodegrades.

In the present invention, the tails 1910 are flat at their ends.. In some embodiments of the invention they may have radiused ends, or may be of any other shape deemed suitable for the implantation site.. The tails 1910 may be of the same shape or of any combination of different shapes deemed appropriate for implantation and anti-migration purposes.

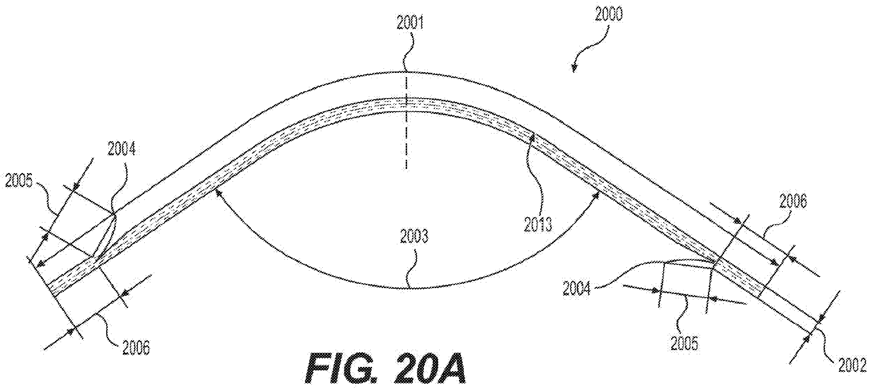

FIG. 20A shows the basic design of another stent 2000 disclosed herein. In one instance, the stent 2000 is made from a polymeric material. In certain instances, the polymeric material may be Aquaprene 8020 with Opaciprene, Dioxaprene 100M with Opaciprene or Lactoprene 7415 with Opaciprene. Appropriate grades of USD, PPD and MDP may also be selected for use in manufacturing the stent. In some instances, the stent comprises a fast-absorbing polymer, such as USD5 (Aquaprene 8020: 20% PEG, 80% p-dioxanone). In other instances, the stent comprises a medium-absorbing polymer, such as PPD3 (Dioxaprene 100M: Poly(para-diaxanone). In still other instances, the stent comprises a slow-absorbing polymer, such as MDP3 (Lactoprene 7415: 74/15/11 copolymer of lactide/trimethylene carbonate/caprolactone). In some instances, the stent is impregnated with a ~1% to -40% BaSCri solution in a suitable carrier. In further instances, the stent is impregnated with a -10% to -30% BaSCri solution in a suitable carrier. In still further instances, the stent is impregnated with a -12% to -22% BaSCri solution in a suitable carrier. In particular instances, the stent is impregnated with a -17% BaSCri solution in a suitable carrier.

The stent length 2001 is variable, dependent upon the application or location the stent 2000 is to be used in. In some instances, the stent length 2001 can be between about 20 mm and about 300 mm. In particular instances, the stent length 2001 is about 40, 60, 80, 100, 120, 150, 225 or 250mm. In some instances, the stent 2000 has an outer diameter 2002 of between about 2.0 mm and about 3.2 mm. In other instances, the stent 2000 has an outer diameter 2002 of between about 2.34 mm and about 2.86 mm. In still other instances, the stent 2000 has an outer diameter 2002 of between about 2.5 mm and about 2.7 mm. In particular instances, the stent 2000 has an outer diameter 2002 of about 2.6 mm +/- 0.26 mm. In more particular instances, the stent 2000 has an outer diameter 2002 of about 2.6 mm +/- 0.13 mm. In some instances, the ends of the stent 2000 are tapered to be narrower than the main body of the stent 2000. The "outer diameter" refers to the linear distance between the two farthest points on the device along a straight line that passes through the center of the device in a cross-section.

The stent 2000 of this instance is flexible. In some instances, the stent flexes after placement in the target location. In some instances, the body of the stent can tolerate a bend 2003 of between about 90° and about 135° without experiencing a degradation of fluid flow rate. In other instances, the body of the stent can tolerate a bend 2003 of between about 100° and about 125° without experiencing a degradation of fluid flow rate. In still other instances, the body of the stent can tolerate a bend 2003 of about 112° without experiencing a degradation of fluid flow rate.

In some instances, the body of the stent 2000 is curved, having a curvature 2013 with a radius of between about 10 mm and about 70 mm. In other instances, the body of the stent 2000 has a curvature 2013 with a radius of between about 20 mm and about 60 mm. In still other instances, the body of the stent 2000 has a curvature 2013 with a radius of between about 30 mm and about 50 mm. In particular instances, the body of the stent 2000 has a curvature 2013 with a radius of about 40 mm.

The stent 2000 comprises two anti-migration devices 2004 that expand outwards from the elongated body of the stent 2000 so as to anchor the stent in position within a bodily lumen. In some instances, the anti-migration devices are elongated protrusions that extend from the stent and contact the tissue of the lumen in order to hold the stent in place. In some instances, the tip of the anti-migration devices is pointed, so that the tip can embed in the tissue. In some instances, the anti-migration devices are held flush with the surface of the stent prior to and during deployment and are allowed to fold out following placement at the target site. The anti-migration devices 2004 are placed in proximity to the ends of the stent, however, one of ordinary skill will understand that the placement of the anti-migration devices 2004 is not limiting on the application. In some instances, the anti-migration devices 2004 are between about 1 mm and about 12 mm in length 2005. In further instances, the anti-migration devices 2004 are between about 3 mm and about 10 mm in length 2005. In still further instances, the anti-migration devices 2004 are between about 5 mm and about 8 mm in length 2005. In a particular instance, the anti-migration devices 2004 are about 7 mm in length 2005. Furthermore, in some instances, the anti-migration devices 2004 are a distance 2006 of between about 1 mm and about 12 mm from each end of the stent 2000. In further instances, the anti-migration devices 2004 are a distance 2006 of between about 3 mm and about 10 mm from each end of the stent 2000. In still further instances, the anti-migration devices 2004 are a distance 2006 of between about 5 mm and about 8 mm from each end of the stent 2000. In a particular instance, the anti-migration devices 2004 are a distance 2006 of about 7 mm from each end of the stent 2000. In some instances, the anti-migration devices 2004 are the same distance 2006 from each end of the stent 2000. In other instances, the anti-migration devices 2004 are different distances 2006 from each end of the stent 2000.

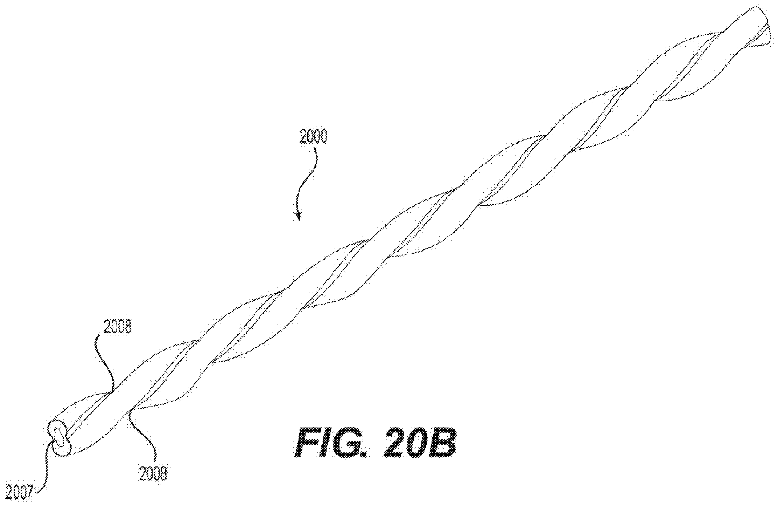

FIG. 20B is a 3-D rendering of the stent 2000. The end of this perspective view shows that the stent has a single central lumen 2007. The longitudinal central lumen 2007 of the stent 2000 is formed within the polymeric material and provides a channel for a guide wire. The narrowing of the stent on either side of the central lumen 2007 creates opposing external channels 2008 for fluid flow on the external surface of the stent 2000. As shown in FIG. 20B, the stent 2000 is twisted, causing the opposing external channels 2008 to spiral around the stent 2000.

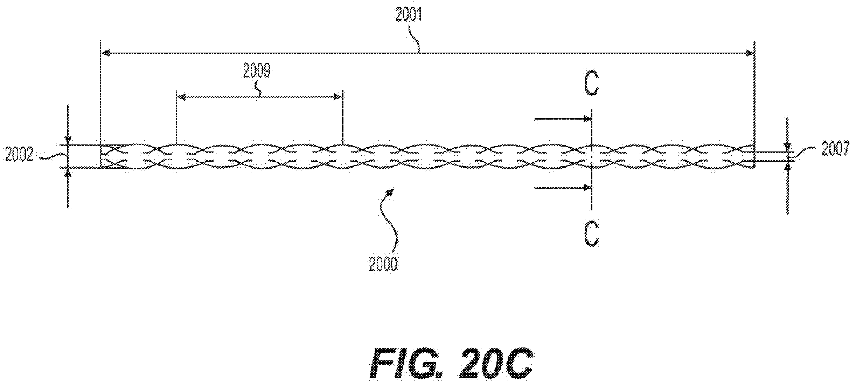

FIG. 20C is a side view of the stent 2000, showing the spiraling of the external channels around the central lumen 2007 (dashed lines). The inventors have surprisingly found that the fluid flow through the external channels is optimized by controlling the number of twists of the stent per inch (i.e., number of turns per 25.4 mm, shown in FIG. 20C as 2009, wherein one twist is a 360 degree rotation around the central axis of the stent). In addition, the present inventors have found that increasing the number of twists per inch (TPI) increases/improves the flexibility of the stent, as measured by deflection of the stent material with an equivalent amount of force applied. Increasing the TPI (for example, from 1 TPI to 2 TPI or from 2 TPI to 3 TPI) increases the amount of deflection, thereby indicating that higher TPI produces a more flexible stent. In some instances, the number of twists is at least, or more than, 1 twist per inch. In other instances, the number of twists is at least, or more than, 1.5 twists per inch. In still other instances, the number of twists is at least, or more than, 1.75 twists per inch. In yet other instances, the number of twists is between about 1.5 and 2.5 twists per inch. In even other instances, the number of twists is between about 1.75 and 2.25 twists per inch. In still other instances, the number of twists is about 2 twists per inch. In particular instances, the number of twists is at least, or more than, 2 twists per inch. In further instances, the number of twists equals 2 twists per inch. In other further instances, the number of twists is more than 2 twists per inch. The twisting of the stent may be carried out by any means known to one of ordinary skill in the art. In some instances, the stent may be formed in a straight, non-twisted, configuration, followed by twisting or machining to the desired number of twists/inch. In other instances, the stent 2000 may be formed or molded in the twisted shape. The polymer material from which the stent 2000 is made may be fixed in place in the twisted position by any means known to one of ordinary skill in the art, e.g., a heating process, etc.

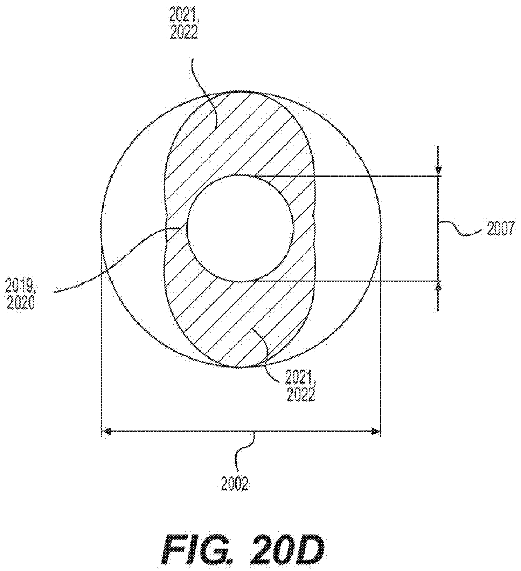

FIG. 20D shows a cross-section view of the stent 2000 at line C-C of FIG. 20C and looking towards one end of the stent 2000. In some instances, the central channel 2007 is between about 0.8 mm and about 1.2 mm in diameter. In other instances, the central channel 2007 is between about 0.9 mm and about 1.1 mm in diameter. In still other instances, the central channel 2007 is between about 0.95 mm and about 1.05 mm in diameter. In particular instances, the central channel 2007 is about 1 mm +/- 0.1 mm in diameter. The "diameter" of the central channel refers to the linear distance between the two farthest points within the lumen of the central channel along a straight line that passes through the center of the central channel in a cross-section.

Still referring to FIG. 20D, the cross-section of the stent comprises a central circle 2019 that surrounds the central channel 2007 and upper and lower bolsters 2021 that overlap the central circle 2019 and, in some instances, each other. The sides of the central circle 2019, between the points where the bolsters 2021 intersect with the central circle 2019, form the minor, thinner, walls 2020 of the stent around the central channel 2007. In some instances, the thickness of the minor walls 2020 is between about 0.1 mm and about 0.3 mm. In other instances, the thickness of the minor walls 2020 is between about 0.15 mm and about 0.25 mm. In particular instances, the thickness of the minor walls 2020 is about 0.2 mm +/- 0.02 mm. In more particular instances, the thickness of the minor walls 2020 is about 0.2 mm +/- 0.01 mm.

Still referring to FIG. 20D, the upper and lower bolsters 2021 are generally elliptical, oval or circular in shape. In the case of an oval or elliptical bolster 2021, the longest longitudinal axis of the bolster 2021 may lie oriented along the main axis of the cross section of the stent, as shown in FIG. 20D. In some instances, the longest longitudinal axis of an oval or elliptical bolster 2021 may he oriented perpendicular to the main axis of the cross section of the stent. The bolsters 2021 form the major, thicker, walls of the stent.

In some instances, the thickness of the major walls 2022 is between about 0.6 mm and about 1.0 mm at the main axis. In other instances, the thickness of the major walls 2022 is between about 0.7 mm and about 0.9 mm at the main axis. In particular instances, the thickness of the major walls 2022 is about 0.8 mm +/- 0.08 mm at the main axis. In more particular instances, the thickness of the major walls 2022 is about 0.8 mm +/- 0.04 mm at the main axis.

In some instances, the bolsters 2021 have a side-to-side thickness of about 1.4 mm +/- 0.14 mm. In more particular instances, the bolsters 2021 have a side-to-side thickness of about 1.4 mm +/- 0.07 mm.

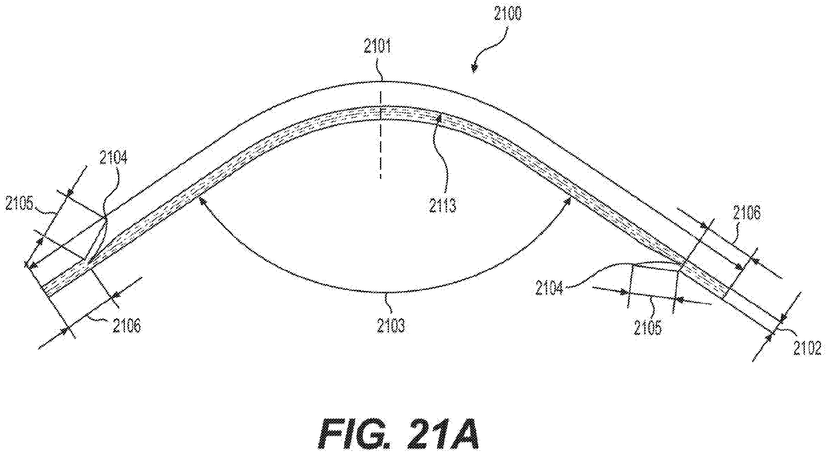

FIG. 21 A shows the basic design of another stent 2100 disclosed herein. In one instance, the stent 2100 is made from a polymeric material. In certain instances, the polymeric material may be Aquaprene 8020 with Opaciprene, Dioxaprene 100M with Opaciprene, or Lactoprene 7415 with Opaciprene. Appropriate grades of USD, PPD and MDP may also be selected for use in manufacturing the stent. In some instances, the stent comprises a fast-absorbing polymer, such as USD5 (Aquaprene 8020: 20% PEG, 80% p-dioxanone). In other instances, the stent comprises a slow-absorbing polymer, such as PPD3 (Dioxaprene 100M: Poly(para-diaxanone). In still other instances, the stent comprises a slow-absorbing polymer, such as MDP3 (Lactoprene 7415: 74/15/11 copolymer of lactide/trimethylene carbonate/caprolactone). In some instances, the stent is impregnated with a ~1% to -40% BaSCri solution in a suitable carrier. In further instances, the stent is impregnated with a -10% to -30% BaSCri solution in a suitable carrier. In still further instances, the stent is impregnated with a -12% to -22% BaSCri solution in a suitable carrier. In particular instances, the stent is impregnated with a -17% BaSCri solution in a suitable carrier.

The stent length 2101 is variable, dependent upon the application or location the stent 2100 is to be used in. In some instances, the stent length 2101 can be between about 20 mm and about 300 mm. In particular instances, the stent length 2101 is about 40, 60, 80, 100, 120, 150, 200 or 225 mm. In some instances, the stent 2100 has an outer diameter 2102 of between about 2.5 mm and about 5 mm. In other instances, the stent 2100 has an outer diameter 2102 of between about 3.06 mm and about 3.74 mm. In still other instances, the stent 2100 has an outer diameter 2102 of between about 3.3 mm and about 3.5 mm. In particular instances, the stent 2100 has an outer diameter 2102 of about 3.4 +/- 0.34 mm. In more particular instances, the stent 2100 has an outer diameter 2102 of about 3.4 +/- 0.17 mm. In some instances, the ends of the stent 2100 are tapered to be narrower than the main body of the stent 2100. The "outer diameter" refers to the linear distance between the two farthest points on the device along a straight line that passes through the center of the device in a cross-section.

The stent 2100 of this instance is flexible. Upon final placement of the stent 2100, in some instances, the body of the stent can tolerate a bend 2103 of between about 90° and about 135° without experiencing a degradation of fluid flow rate. In other instances, the body of the stent can tolerate a bend 2103 of between about 100° and about 125° without experiencing a degradation of fluid flow rate. In still other instances, the body of the stent can tolerate a bend 2103 of about 112° without experiencing a degradation of fluid flow rate.

In some instances, the body of the stent 2100 is curved, having a curvature 2113 with a radius of between about 10 mm and about 70 mm. In other instances, the body of the stent 2100 has a curvature 2013 with a radius of between about 20 mm and about 60 mm. In still other instances, the body of the stent 2100 has a curvature 2113 with a radius of between about 30 mm and about 50 mm. In particular instances, the body of the stent 2100 has a curvature 2113 with a radius of about 40 mm.

The stent 2100 comprises two anti-migration devices 2104 that expand outwards from the elongated body of the stent 2100 so as to anchor the stent in position within a bodily lumen. The anti-migration devices 2104 are placed in proximity to the ends of the stent, however, one of ordinary skill will understand that the placement of the anti-migration devices 2104 is not limiting on the application. In some instances, the anti-migration devices 2104 are between about 1 mm and about 12 mm in length 2105. In further instances, the anti-migration devices 2104 are between about 3 mm and about 10 mm in length 2105. In still further instances, the anti-migration devices 2104 are between about 5 mm and about 8 mm in length 2105. In a particular instance, the anti-migration devices 2104 are about 7 mm in length 2105. Furthermore, in some instances, the anti-migration devices 2104 are a distance 2106 of between about 1 mm and about 12 mm from each end of the stent 2100. In further instances, the anti-migration devices 2104 are a distance 2106 of between about 3 mm and about 10 mm from each end of the stent 2100. In still further instances, the anti-migration devices 2104 are a distance 2106 of between about 5 mm and about 8 mm from each end of the stent 2100. In a particular instance, the anti-migration devices 2104 are a distance 2106 of about 7 mm from each end of the stent 2100. In some instances, the anti-migration devices 2104 are the same distance 2106 from each end of the stent 2100. In other instances, the anti-migration devices 2104 are different distances 2106 from each end of the stent 2100.

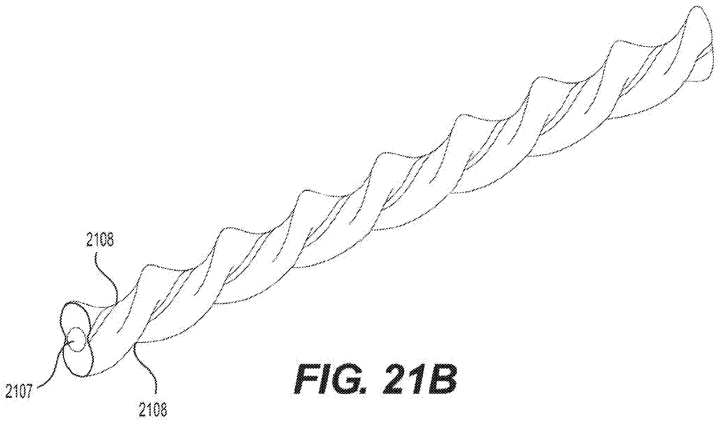

FIG. 21B is a 3-D rendering of the stent 2100. The end of this perspective view shows that the stent has a single central lumen 2107. The longitudinal central lumen 2107 of the stent 2100 is formed within the polymeric material and provides a channel for a guide wire. As shown in FIG. 21B, the stent 2100 is twisted, causing the opposing external channels 2108, created by the narrower side-to-side axis of the stent, to spiral around the stent 2100

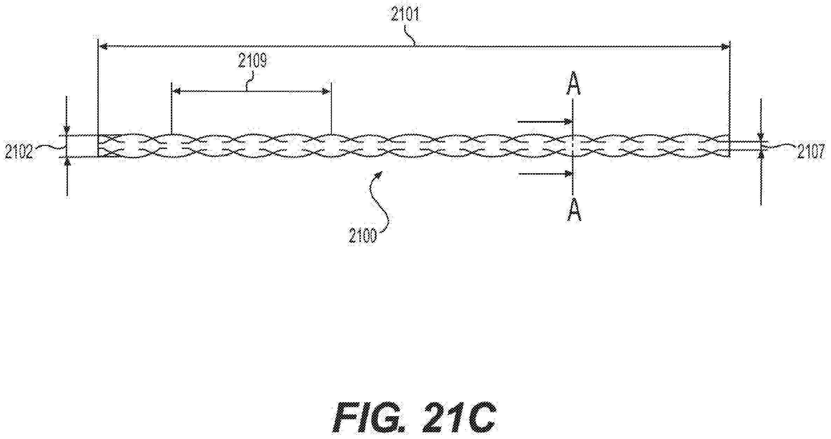

FIG. 21C is a side view of the stent 2100, showing the spiraling of the external channels 2108 around the central lumen 2107 (dashed lines). The inventors have surprisingly found that the fluid flow through the external channels 2108 is optimized by controlling the number of twists of the stent per inch (i.e., number of turns per 25.4 mm, shown in FIG. 21C as 2109, wherein one twist is a 360 degree rotation around the central axis of the stent). In addition, the present inventors have found that increasing the number of twists per inch (TPI) increases/improves the flexibility of the stent, as measured by deflection of the stent material with an equivalent amount of force applied. Increasing the TPI (for example, from 1 TPI to 2 TPI or from 2 TPI to 3 TPI) increases the amount of deflection, thereby indicating that higher TPI produces a more flexible stent. In some instances, the number of twists is at least, or more than, 1 twist per inch. In other instances, the number of twists is at least, or more than, 1.5 twists per inch. In still other instances, the number of twists is at least, or more than, 1.75 twists per inch. In yet other instances, the number of twists is between about 1.5 and 2.5 twists per inch. In even other instances, the number of twists is between about 1.75 and 2.25 twists per inch. In still other instances, the number of twists is about 2 twists per inch. In particular instances, the number of twists is at least, or more than, 2 twists per inch. In further instances, the number of twists equals 2 twists per inch. In other further instances, the number of twists is more than 2 twists per inch. The twisting of the stent may be carried out by any means known to one of ordinary skill in the art. In some instances, the stent may be formed in a straight, non-twisted, configuration, followed by twisting or machining to the desired number of twists/inch. In other instances, the stent 2100 may be formed or molded in the twisted shape. The polymer material from which the stent 2100 is made may be fixed in place in the twisted position by any means known to one of ordinary skill in the art, e.g., a heating process, etc.

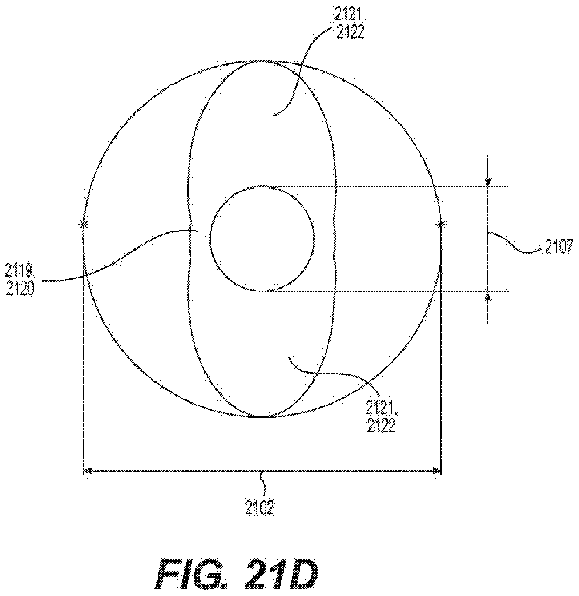

FIG. 21D shows a cross-section view of the stent 2100 at line A- A of FIG. 21 C. In terms of orientation for describing the cross-section, the directions up, down, upper and lower refer direction lying on the main axis that bisects the cross-section of the stent into the longest mirror-image halves. Sides and side-to-side refer to a cross axis that is perpendicular to the main axis. In some instances, the central channel 2107 is between about 0.8 mm and about 1.2 mm in diameter. In other instances, the central channel 2107 is between about 0.9 mm and about 1.1 mm in diameter. In still other instances, the central channel 2107 is between about 0.95 mm and about 1.05 mm in diameter. In particular instances, the central channel 2107 is about 1 mm in diameter. In other particular instances, the central channel 2107 is 1 mm +/- 0.1 mm in diameter. In some instances, the central channel is circular in cross section, in other instances, the central channel is oval or elliptical in cross section. The "diameter" of the central channel refers to the linear distance between the two farthest points within the lumen of the central channel along a straight line that passes through the center of the central channel in a cross-section.

Still referring to FIG. 21D, the cross-section of the stent may comprise a central circle 2119 that surrounds the central channel 2107 and upper and lower bolsters 2121 that may overlap the central circle 2119 and, in some instances, each other. The sides of the central circle 2119, between the points where the bolsters 2121 intersect with the central circle 2119, form the minor, thinner, walls 2120 of the stent around the central channel 2107. In some instances, the thickness of the minor walls 2120 is between about 0.1 mm and about 0.3 mm. In other instances, the thickness of the minor walls 2120 is between about 0.15 mm and about 0.25 mm. In particular instances, the thickness of the minor walls 2120 is about 0.2 mm +/- 0.02 mm. In more particular instances, the thickness of the minor walls 2120 is about 0.2 mm +/- 0.01 mm.

Still referring to FIG. 2 ID, the upper and lower bolsters 2121 are generally elliptical, oval or circular in shape. In the case of an oval or elliptical bolster 2121, the longest longitudinal axis of the bolster 2121 may be oriented along the main axis of the cross section of the stent, as shown in FIG. 2 ID. In some instances, the longest longitudinal axis of an oval or elliptical bolster 2121 may be oriented perpendicular to the main axis of the cross section of the stent. The bolsters 2121 form the major, thicker, walls of the stent.

In some instances, the thickness of the major walls 2122 is between about 0.9 mm and about 1.5 mm at the main axis. In other instances, the thickness of the major walls 2122 is between about 1.0 mm and about 1.4 mm at the main axis. In still other instances, the thickness of the major walls 2122 is between about 1.1 mm and about 1.3 mm at the main axis. In particular instances, the thickness of the major walls 2122 is about 1.2 mm +/- 0.12 mm at the main axis. In more particular instances, the thickness of the major walls 2122 is about 1.2 mm +/- 0.06 mm at the main axis.

In some instances, the bolsters 2121 have a side-to-side thickness of about 1.4 mm +/- 0.14 mm. In more particular instances, the bolsters 2121 have a side-to-side thickness of about 1.4 mm +/- 0.07 mm.

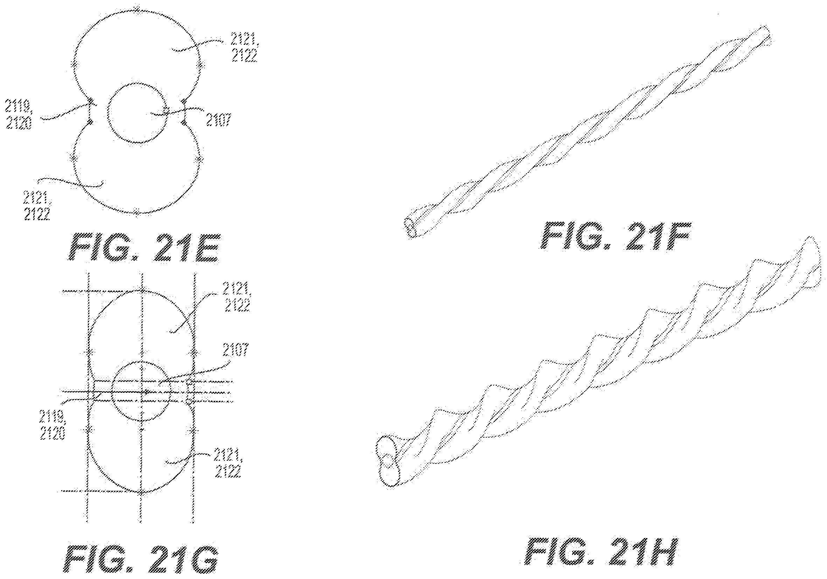

FIG. 21E shows an alternative cross-section for an instance of the stent 2100 and FIG. 21 F shows the corresponding 3-D view. In some instances, the bolsters 2121 have a side-to-side thickness of between about 1.5 mm and about 1.9 mm. In other instances, the bolsters 1921 have a side-to-side thickness of between about 1.6 mm and about 1.8 mm. In particular instances, the bolsters 1921 have a side-to-side thickness of about 1.7 mm +/- 0.17 mm. In more particular instances, the bolsters 1921 have a side-to-side thickness of about 1.7 mm +/- 0.085 mm.

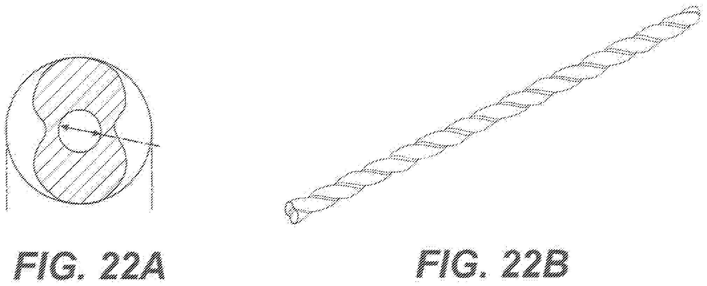

FIG. 21G shows another alternative cross-section for an instance of the stent 2100 and FIG. 21H shows the corresponding 3-D view. In some instances, the bolsters 2121 have a side-to-side thickness of between about 1.4 mm and about 1.5 mm. In particular instances, the bolsters 2121 have a side-to-side thickness of about 1.45 mm +/- 0.145 mm. In more particular instances, the bolsters 2121 have a side-to-side thickness of about 1.45 mm +/- 0.07 mm. FIG. 22A shows an exemplary alternative feature for the stents described in FIGS. 19A through 21H, wherein the junctions between the central circle and the bolsters are tapered or rounded. FIG. 22B is a 3-D rendering of the exemplary stent of FIG. 22A.

A biodegradable stent of the present application is useful for the treatment or palliation of strictures of a lumen in a subject in need thereof. In some embodiments, the lumen is a duct. In some further embodiments, the duct is a bile duct. In some still further embodiments, the bile duct is a hepatic, cystic, common bile or pancreatic duct. In some embodiments, the stricture is caused by, co-occurring with or related to a malignancy or a benign disease or condition of the liver, pancreas, duodenum, gall bladder or biliary tree. A biodegradable stent of the present application provides less complications in a subject, does not require costly removal procedures, has a lower clinical cost because it does not need sequential replacement and reduces loss of work time for the subject in need thereof.

In some embodiments, a biodegradable stent of the present application degrades by hydrolysis. In some embodiments, degradation of the biodegradable stent occurs on the outer surfaces, wherein the outer layer is degraded off and the stent progressively degrades from the outside towards the center.

A stent of the present application is capable of opening the lumen of a duct and allows bile to drain away. The present stent is biocompatible according to ISO 10993. The present stent is capable of withstanding compression without obstructing a duct. The present stent is loadable into a duodenal scope. The flexibility and column strength of the present stent is high enough to push the stent from the scope into a duct and can be deployed in a target location, and is visible, under fluoroscopy. A stent of the present application is insertable into a duct without perforating or otherwise damaging the duct. The ends of the stent minimize tissue granulation and the stent has high friction to prevent migration of the stent from the target location, while being capable of repositioning after deployment. The present stent is removable after implantation without damage to the tissue of the lumen. A stent of the present invention has a shelf life of 1 to 2 years after sterilization.

The present stent is typically made from a polymer material, plastics, metals, or alloys. Notable variations exist within each type. In certain embodiments, the stent is made from a non-polymer material. Examples of such materials include, but are not limited to, stainless steel, cobalt alloys such as cobalt-chromium, titanium alloys, tantalum, niobium, tungsten, molybdenum and nitinol. For example, self-expanding metal stents are generally made from nitinol, while some balloon-expandable metal stents are made from stainless steel. A coating, such as polyurethane coating, may be used to prevent non-polymer stent material from coming into direct contact with its surroundings. The coating slows down the rate of in growth, allowing the stent to remain in the patient with a lower potential for side effects.

The stent may also be made with a bioabsorbable material. Examples of bioabsorbable materials include, but are not limited to, polylactic acid or polylactide (PLA), poly glycolic acid or polyglycolide (PGA), poly-s-caprolactone (PCL), polyhydroxybutyrate (PHB), polyethylene glycol (PEG), p-dioxanone, poly-(p-dioxanone) (PPDO), trimethylene carbonate, caprolactate and co-polymers thereof.

In one embodiment, the bioabsorbable material is degraded based on varying levels of pH. For example, the material may be stable at a neutral pH but degrades at a high pH. Examples of such materials include, but are not limited to chitin and chitosan. In another embodiment, the bioabsorbable material is degradable by enzymes, such as lysozymes.

In another embodiment, the polymers include transparent plastic polymers, thermoplastic polyurethane or silicone polymers.

In another embodiment, the elongated body comprises a combination of a polymer and a non-polymer material.

In another related embodiment, the elongated stent body is made of a magnesium and chitin alloy.

In another related embodiment, the elongated stent body is made with a magnesium core coated with a chitin chitosan, N-acylchitosan hydrogel outer layer. The magnesium core may additionally include rare earth materials.

In another related embodiment, the elongated stent body is made of a chitin and chitosan, N-acylchitosan hydrogel and magnesium alloy with raw earth elements.

In another embodiment, the bioabsorbable material may absorb moisture and expand in situ at the treatment site. For example, the stent made of chitin or a variable copolymer of chitin and PLGA or chitin and magnesium and other rare earth minerals would swell once it comes into contact with various body fluids. In one embodiment, the stent has a pre-implantation diameter D_{prc} (i.e., dry diameter) of 2.8 mm and is expandable to a post implantation diameter Dₚₒₛₜ , (i.e., wet diameter) of 3.3 mm after exposure to body liquid in a lumen. As used hereinafter, the "pre-implantation diameter Dₚᵣₑ " refers to the largest diameter of a stent body before implantation and the "post-implantation diameter Dₚₒₛₜ " refers to the largest diameter of the stent body after implantation.

In some embodiments, the stent is made of a fast-absorbing bioabsorbable material that degrades within about two to four weeks. In particular embodiments, the fast absorbing bioabsorbable material is a mixture or combination of PEG and p-dioxanone. In a further embodiment, PEG comprises about 10-30% and p-dioxanone comprises about 70- 90% of the mixture or combination. In a still further embodiment, PEG comprises about 15- 25% and p-dioxanone comprises about 75-85% of the mixture or combination. In an even further embodiment, PEG comprises about 12-22% and p-dioxanone comprises about 78- 82% of the mixture or combination. In a yet further embodiment, PEG comprises about 20% and p-dioxanone comprises about 80% of the mixture or combination.

In some embodiments, the stent is made of a medium-absorbing bioabsorbable material that degrades within about three to six weeks. In particular embodiments, the medium-absorbing bioabsorbable material is PPDO, or a copolymer thereof.

In some embodiments, the stent is made of a medium-to-slow-absorbing bioabsorbable material that degrades within about six week to four months.

In some embodiments, the stent is made of a slow-absorbing bioabsorbable material that degrades within about four to six months. In particular embodiments, the fast absorbing bioabsorbable material is a copolymer of lactide, trimethylene carbonate and caprolactate. In particular embodiments, the copolymer comprises a percentage composition of PLA/trimethylene carbonate/caprolactate that is about 70-80/10-20/5-15, respectively. In further embodiments, the copolymer comprises a percentage composition of PLA/trimethylene carbonate/caprolactate that is about 72-76/13-17/9-13, respectively. In still further embodiments, the copolymer comprises a percentage composition of PLA/trimethylene carbonate/caprolactate that is about 74/15/11, respectively.

In some embodiments, the bioabsorbable material is coated or impregnated with a biocompatible radio-opaque substance to aid in visualization of the stent during or after emplacement, for example by fluoroscopy or x-ray. In some embodiments, the radio opaque substance is a BaSCri solution. In further embodiments, the solution comprises about 10-25% BaSCri. In still further embodiments, the solution comprises about 12-22% BaSCri. In even further embodiments, the solution comprises about 17% BaSC In some embodiments, the radio-opaque substance comprises metal particles. In further embodiments, the particles are nanoparticles. In exemplary non-limiting embodiments, the metal comprises tantalum.

In some embodiments, the stent is a biliary stent with a diameter of 5F to 12F and a length of 10-180 mm. In some embodiments, the stent is a pancreatic stent with a diameter of 3F to 11.5F and a length of 20-250 mm. In some embodiments, the biliary or pancreatic stent is a self-expanding metal stent. In some embodiments, the biliary or pancreatic stent is made of a polymer material. In some embodiments, the biliary or pancreatic stent is constructed to have a minimal strength retention of 12 days, 25 days or 12 weeks, where strength retention is defined by the presence of at least 10% of the initial strength parameter (e.g. the stent remains intact with no breaks, tested in a simulated degradation model). In some embodiments, the biliary or pancreatic stent contains by weight 16.8% PEG, 67.2% p-dioxanone (in the form of a 20%/80% PEG/p-dioxanone coploymer) and 16% BaS04 (barium sulfate), and has minimal strength retention of 12 days. In some embodiments, the biliary or pancreatic stent contains by weight 84% Poly(para-dioxanone) and 16% BaS04, and has minimal strength retention of 25 days. In some embodiments, the biliary or pancreatic stent contains by weight 62.16% lactide, 12.6% trimethylene carbonate, 9.24% caprolactone (in the form of a 74%/ 15 %/ 11% coploymer) and 16% BaS04, and has a has minimal strength retention of 12 weeks.

In some embodiments, the biliary stent experiences initial surface degradation upon implantation, which allows for bile cleansing. In some embodiments, the biliary stent comprises a double-channel helical twist on the exterior surface to allow bile flow on the outside of the stent. In comparison to other stents, the biliary stent of the present application provides better simulated flow rates, better simulated migration resistance and better crush resistance.

In another embodiment, the bioabsorbable material is embedded with, or configured to carry, various agents or cells. The agents may be coupled to the outer and/or inner surfaces of stent body or integrated into the bioabsorbable material itself. In one embodiment, the bioabsorbable stent has a hollow center lumen so that agents may be placed inside the lumen to increase the dose release. The stent can additionally have multiple reservoirs, one inside the other, so that when the outer layer is absorbed the next reservoir is exposed and a further release of a larger dose of the chosen agents or cells. The chosen agent or cells may also be mixed with the polymer for sustained release.

Examples of agents that can be embedded into or carried by a stent include, but are not limited to, small molecule drugs, biologicals and gene transfer vectors. Examples of small molecule drugs include, but are not limited to, sirolumus, rapamycian, and other anti-proliferating agent.

Examples of biologicals include, but are not limited to, antimicrobial agents and chemotherapeutic agents.

The term "antimicrobial agent" as used in the present application means antibiotics, antiseptics, disinfectants and other synthetic moieties, and combinations thereof, that are soluble in organic solvents such as alcohols, ketones, ethers, aldehydes, acetonitrile, acetic acid, formic acid, methylene chloride and chloroform. Classes of antibiotics that can possibly be used include tetracyclines (i.e., minocycline), rifamycins (i.e.. rifampin), macrolides (i.e., erythromycin), penicillins (i.e.. nafcillin), cephalosporins (i.e.. cefazolin), other beta-lactam antibiotics (imipenem, aztreonam), aminoglycosides (i.e.. gentamicin), chloramphenicol, sulfonamides (i.e., sulfamethoxazole), glycopeptides (i.e.. vancomycin), quinolones (i.e.. ciprofloxacin), fusidic acid, trimethoprim, metronidazole, clindamycin, mupirocin, polyenes (i.e., amphotericin B), azoles (i.e., fluconazole) and beta-lactam inhibitors (i.e.. sulbactam).

Examples of specific antibiotics that can be used include minocycline, rifampin, erythromycin, nafcillin, cefazolin, imipenem, aztreonam, gentamicin, sulfamethoxazole, vancomycin, ciprofloxacin, trimethoprim, metronidazole, clindamycin, teicoplanin, mupirocin, azithromycin, clarithromycin, ofloxacin, lomefloxacin, norfloxacin, nalidixic acid, pefloxacin, pefloxacin, amifloxacin, enoxacin, fleroxacin, temafloxacin, tosufloxacin, clinafloxacin, sulbactam, clavulanic acid, amphotericin B, fluconazole, itraconazole, ketoconazole and nystatin. Other examples of antibiotics, such as those listed in U.S. Pat. No. 4,642,104, will readily suggest themselves to those of ordinary skill in the art. Examples of antiseptics and disinfectants are thymol, a- terpineol, methylisothiazolone, cetylpyridinium, chloroxylenol, hexachlorophene, cationic biguanides (i.e.. chlorhexidine, cyclohexidine), methylenechloride, iodine and iodophores (i.e., povidone-iodine), triclosan, firanmedical preparations (i.e.. nitrofurantoin, nitrolurazone), methenamine, aldehydes (i.e.. glutaraldehyde, formaldehyde) and alcohols. Other examples of antiseptics and disinfectants will readily suggest themselves to those of ordinary skill in the art.

The stent of the present application may also be prepared with antimicrobial agents in other ways customary in the art. For example, the stent may be made in its entirety or in part of an antimicrobial polymer, or at least one surface of the stent may have embedded, by ion beam assisted deposition or co-extrusion techniques, therein with atoms of an antimicrobial polymer. Other suitable examples can be found in the art, for example, U.S. Pat. No. 5,520,664.

Chemotherapeutic agents can be coupled with the stent of the present application in a manner analogous to that of antimicrobial agents. Exemplary chemotherapeutic agents include but are not limited to cis-platinum, paclitaxol, 5- fluorouracil, gemcytobine and navelbine. The chemotherapeutic agents are generally grouped as DNA-interactive agents, antimetabolites, tubulin-interactive agents, hormonal agents, hormone-related agents, and others such as asparaginase or hydroxyurea. Each of the groups of chemotherapeutic agents can be further divided by type of activity or compound. The chemotherapeutic agents used in combination with the anti-cancer agents or benzimidazoles of this application include members of all of these groups. For a detailed discussion of the chemotherapeutic agents and their method of administration, see Dorr, et al, Cancer Chemotherapy Handbook, 2d edition, pages 15-34, Appleton & Lange (Connecticut, 1994).

Examples of DNA-Interactive agents include, but are not limited to, alkylating agents, DNA strand-breakage agents; intercalating and nonintercalating topoisomerase II inhibitors, and DNA minor groove binders. Alkylating agents generally react with a nucleophilic atom in a cellular constituent, such as an amino, carboxyl, phosphate, or sulfhydryl group in nucleic acids, proteins, amino acids, or glutathione. Examples of alkylating agents include, but are not limited to, nitrogen mustards, such as chlorambucil, cyclophosphamide, isofamide, mechlorethainine, Melphalan, uracil mustard; aziridines, such as thiotepa; methanesulfonate esters such as busulfan; nitroso, ureas, such as cannustine, lomustine, streptozocin; platinum complexes, such as cisplatin, carboplatin; bioreductive alkylator, such as mitomycin, and procarbazine, dacarbazine and altretamine. DNA strand breaking agents include, but are not limited to, bleomycin. Intercalating DNA topoisomerase II inhibitors include, but are not limited to, intercalators such as amsacrine, dactinomycin, daunorubicin, doxorubicin, idarubicin, and mitoxantrone.

Nonintercalating DNA topoisomerase II inhibitors include, but are not limited to etoposide and teniposide. DNA minor groove binders include, but are not limited to, plicamycin.

Antimetabolites interfere with the production of nucleic acids by one or the other of two major mechanisms. Some of the drugs inhibit production of the deoxyribonucleoside triphosphates that are immediate precursors for DNA synthesis, thus inhibiting DNA replication. Some of the compounds, for example, purines or pyrimidines, are sufficient to be able to substitute for them in the anabolic nucleotide pathways. These analogs can then be substituted into the DNA and RNA instead of their normal counterparts. The antimetabolites useful herein include: folate antagonists such as methotrexate and trimetrexate pyrimidine antagonists, such as fluorouracil, fluorodeoxyuridine, CB3717, azacytidine, cytarabine, and floxuridine purine antagonists include mercaptopurine, 6- thioguanine, fludarabine, pentostatin; sugar modified analogs include cyctrabine, fludarabine; ribonucleotide reductase inhibitors include hydroxyurea. Tubulin interactive agents act by binding to specific sites on tubulin, a protein that polymerizes to form cellular microtubules. Microtubules are critical cell structure units. When the interactive agents bind on the protein, the cell cannot form microtubules tubulin interactive agents including vincristine and vinblastine, both alkaloids and paclitaxel.

Hormonal agents are also useful in the treatment of cancers and tumors. They are used in hormonally susceptible tumors and are usually derived from natural sources. These include: estrogens, conjugated estrogens and ethinyl estradiol and diethylstilbestrol, chlorotrianisene and idenestrol; progestins such as hydroxyprogesterone caproate, medroxyprogesterone, and megestrol; androgens such as testosterone, testosterone propionate; fluoxymesterone, metbyltestosterone; adrenal corticosteroids are derived from natural adrenal cortisol or hydrocortisone. They are used because of their anti-inflammatory benefits as well as the ability of some to inhibit mitotic divisions and to halt DNA synthesis. These compounds include prednisone, dexamethasone, methylprednisolone, and prednisolone.

Hormone-related agents include, but are not limited to, leutinizing hormone releasing hormone agents, gonadotropin-releasing hormone antagonists and anti-hormonal agents. Gonadotropin-releasing hormone antagonists include leuprolide acetate and goserelin acetate. They prevent the biosynthesis of steroids in the testes and are used primarily for the treatment of prostate cancer.

Antihormonal agents include antiestrogenic agents such as tamosifen, antiandrogen agents such as Flutamide; and antiadrenal agents such as mitotane and aminoglutethimide. Hydroxyurea appears to act primarily through inhibition of the enzyme ribonucleotide reductase. Asparaginase is an enzyme that converts asparagine to nonfunctional aspartic acid and thus blocks protein synthesis in the tumor.

Gene transfer vectors are capable of introducing a polynucleotide into a cell. The polynucleotide may contain the coding sequence of a protein or a peptide, or a nucleotide sequence that encodes a iRNA or antisense RNA. Examples of gene transfer vectors include, but are not limited to, non-viral vectors and viral vectors. Non-viral vectors typically include a plasmid having a circular double stranded DNA into which additional DNA segments can be introduced. The non-viral vector may be in the form of naked DNA, poly cationic condensed DNA linked or unlinked to inactivated virus, ligand linked DNA, and liposome- DNA conjugates. Viral vectors include, but are not limited to, retrovirus, adenovirus, adeno- associated virus (AAV), herpesvirus, and alphavirus vectors. The viral vectors can also be astrovirus, coronavirus, orthomyxovirus, papovavirus, paramyxovirus, parvovirus, picomavirus, poxvirus, or togavirus vectors.

The non-viral and viral vectors also include one or more regulatory sequences operably linked to the polynucleotide being expressed. A nucleotide sequence is "operably linked" to another nucleotide sequence if the two sequences are placed into a functional relationship. For example, a coding sequence is operably linked to a 5' regulatory sequence if the 5' regulatory sequence can initiate transcription of the coding sequence in an in vitro transcription/translation system or in a host cell. "Operably linked" does not require that the DNA sequences being linked are contiguous to each other. Intervening sequences may exist between two operably linked sequences.

In one embodiment, the gene transfer vector encodes a short interfering RNA (siRNA). siRNAs are dsRNAs having 19-25 nucleotides. siRNAs can be produced endogenously by degradation of longer dsRNA molecules by an RNase Ill-related nuclease called Dicer. siRNAs can also be introduced into a cell exogenously or by transcription of an expression construct. Once formed, the siRNAs assemble with protein components into endoribonuclease-containing complexes known as RNA-induced silencing complexes (RISCs). An ATP-generated unwinding of the siRNA activates the RISCs, which in turn target the complementary mRNA transcript by Watson-Crick base-pairing, thereby cleaving and destroying the mRNA. Cleavage of the mRNA takes place near the middle of the region bound by the siRNA strand. This sequence specific mRNA degradation results in gene silencing. In another embodiment, the gene transfer vector encodes an antisense RNA.

Examples of cells include, but are not limited to, stem cells or other harvested cells.

### Manufacture of Stent with Anti-Migration Extensions

The stent body and surface channels can be laser cut, water jet cut, extruded, stamped, molded, lathed or formed. In one embodiment, the stent is cut from a single polymer tube that may be extruded. The tube may be hollow or the center may be cored out at varying diameters suitable for the particular indication.

The stent is then etched and is formed on a suitable shaping device to give the stent the desired external geometry. Both the synthetic collar techniques and in vitro valuation techniques show the remarkable ability of stents of the present application to convert acting force into deformation work absorbed by the angled structure, which prevents excessive scaffolding stress, premature material fatigue and accelerated obsolescence.

The stent of the present application may be formed in such a way as to allow fluid flow to change in the pitch of the flow to improve flow dynamics and to speed the flow of fluids throughout the device. From a tight radial design to a more longitudinal design.

In one embodiment spiral surface channels with large cross-section areas are formed to accommodate large volumes of body fluid. In another embodiment, multiple channels with small cross-section area are formed to accommodate large volumes of body fluid. In another embodiment, the stent body contains a large center lumen to allow for fluid flow and a plurality of small cross-section area channels on the surface to stabilize the stent in vivo.

In another embodiment, the lips of the channel walls are taped to increase the surface area for fluid flow and grip. Changes in the depth of the pitch of the channels will also have an impact on fluid flow and stability.

In one embodiment, the stent is formed on a shaping tool that has substantially the desired contour of the external stent dimensions. In the event the stent is to be shaped to the dimensions of a particular lumen, optical photography and/or optical videography of the target lumen may be conducted prior to stent formation. The geometry of corresponding zones and connector regions of the stent then can be etched and formed in accordance with the requirements of that target lumen. In particular, if the topography of the biliary duct of a particular patient is captured optically and the appropriate dimension provided, a patient specific prosthesis can be engineered. These techniques can be adapted to other non-vascular lumens but is very well suited for vascular applications where patient specific topography is a function of a variety of factors such as genetics, lifestyle, etc.

Unlike stents made from shape memory metals, the stents of the present application can take on an infinite number of characteristic combinations as zones and segments within a zone can be modified by changing angles, segment lengths, segment thicknesses, pitch during the etching and forming stages of stent engineering or during post formation processing and polishing steps. Moreover, by modifying the geometry, depth, and diameter of the channels between zones, additional functionality may be achieved, such as flexibility, increased fluid transport, and changes in friction.

The stent body comprises at least two anti-migration tails extending from at least one end of the body of the stent. Moreover, the stent body may be flared out for improved anti-migration capability, and the flared end can be extended into one or more flapping flares, thereby forming one or more stent tails

In certain examples, the lateral flaps or the longitudinal tails are added to the stent body by extrusion. In some embodiments, the lateral flaps or the longitudinal tails are created in a preexisting mold. In some embodiments, the lateral flaps or the longitudinal tails are laser cut out of the lateral wall of the stent body. In some embodiments, the flaps or the tails are attached to the stent body by an adhesive, or by any other means considered appropriate.

In other embodiments, the lateral flaps or the longitudinal tails are molded into a variety of shapes. In one instance disclosed herein, the lateral flaps or the longitudinal tails are tapered into conical shape, and in another example, the flaps and the tails are ridge-shaped. In one embodiment, the tips of the flaps and tails are radiused, and in another embodiment the tips of the flaps and tails are machined to be pointed in order to decrease the contact area with the surrounding lumen tissue, and thereby immobilize the stent in the lumen.

In some examples, the lateral flaps or the longitudinal tails are pulled towards the stent body and tucked in, in preparation for transportation to a target site. In other embodiments, the lateral flaps or the longitudinal tails are wrapped together with a shell and aligned with the stent body in preparation for transportation. In one embodiment, the shell is made to slide off of the flaps or the tails upon being pulled. In another embodiment, the shell is made out of a biodegradable material. In one embodiment, the shell dissolves in order to release the flaps or the tails, and to, consequently, enable anchoring of the flaps or the tails.

### Use of Stent with Anti-Migration Extensions

A stent of the present application is used to support a target site in a vessel, duct or lumen and optimize the flow of bodily fluids in a subject in need thereof. Following identification of the target site, an entry portal is established into a vessel, duct or lumen leading to the target site. A guide wire is advanced through the entry portal and vessel, duct or lumen to or through the target site. The stent is then pushed along the guide wire until it reaches the target site and is emplaced there, followed by the withdrawal of the guide wire.

In one example, the anti-migratory flaps or tails are tucked during deployment, in order to facilitate transportation of the stent to the target site. For example, the lateral flaps or the longitudinal tails may be wrapped together with a shell and aligned with the stent body in preparation for transportation. In certain embodiments, the shell slides off of the flaps or the tails upon being pulled, and the flaps or the tails unfold to be deployed. In another embodiment, the shell dissolves in order to release the flaps or the tails. Subsequently, the flaps or the tails anchor into the surrounding tissue to prevent stent migration.

In one embodiment, the support device is transported through an endoscope in order to visualize the transportation and the placement of the device. In one embodiment, the flaps or the tails are pushed to be oriented downward in the endoscope, i.e., in the direction of advancing the support device. During the transportation, in one example the flaps or the tails remain in the downward position. The number of flaps/tails may vary, and the support device may include, two, three or more flaps/tails on one or both of its ends. In the invention, two flaps/tails are in a Y-shape relative to the stent body, and in another example, three flaps/tails are in a three-legged stool arrangement relative to the stent body.

Once the support device arrives at the placement site, the flaps or the tails expand laterally outward and anchor into the surrounding tissue to prevent migration. In one embodiment, the stent includes the flaps/tails only on one end, distal or proximal relative to the stent advancing direction. In another embodiment, the stent includes the flaps/tails on both distal and proximal ends. In yet another embodiment, the distal flaps/tails going into the body first prevent migration out of the organ, and in another embodiment the proximal flaps/tails prevent migration into the organ. In an embodiment when the stent is, for example, in a kidney to allow flow into the bladder, the Y-shaped flaps/tails can be on both ends to prevent the stent from coming out of the kidney.

### Kit

Another teaching of the present disclosure relates to a kit. The kit comprises at least one stent of the present application. In some instances, the kit further comprises a guide wire for emplacing the stent at a target location. In some instances, the kit further comprises a pushing catheter for moving the stent along the guide wire. In some instances, the kit comprises an introducer sheath or introducer tube. In some instances, the kit further comprises a cannula. In some instances, the kit further comprises a sphincterotome. In some instances, the kit comprises a radio-opaque dye.

### EMBODIMENTS

The present invention relates to a stent comprising: an elongated body composed of a biodegradable material having a proximal end, a distal end, at least one open channel formed on the exterior surface of said body to provide fluid communication between said proximal end and said distal end, a central lumen open at the proximal and distal ends of the stent for the passage of a guide wire, and wherein said stent further comprises at least two anti-migration tails extending from at least one end of the elongated body, wherein the at least two anti-migration tails extending from at least one end of the elongated body form a Y-shape with the elongated body and wherein said anti-migration tails are flat-shaped at their ends.

The present application also relates to a stent that comprises at least one lateral anti-migration device and at least two anti-migration tails extending from at least one end of the elongated body. The stent comprises at least two anti-migration tails extending from at least one end of the elongated body. The at least two anti-migration tails extending from at least one end of the elongated body may form Y-shape with the elongated body.

In some embodiments, the at least two anti-migration tails extending from at least one end of the elongated body may be different in length and/or may be different in thickness. In one embodiment, the at least two anti-migration tails extending from at least one end of the elongated body are made of different material than the elongated body. Length, thickness, and/or flexibility of the at least two anti-migration tails may be determined based on expected dimensions of a target site for stent placement relative to dimensions of the stent.

Disclosed herein, but not claimed, the shape of the at least two anti-migration tails extending from at least one end of the elongated body is tapered. In the invention the shape of the at least two anti-migration tails extending from at least one end of the elongated body is flat.

In another example, the tips of the at least two anti-migration tails extending from at least one end of the elongated body are radiused.

Disclosed herein, but not claimed, is also a method of immobilizing a stent in a subject in need thereof, comprising: establishing an entry portal into a vessel, duct or lumen contiguous with a target site for stent placement, advancing a guide wire through the entry portal and said vessel, duct or lumen contiguous to said target site, advancing said stent along said guide wire to said target site, said stent comprising an elongated body composed of a bioabsorbable polymer having a proximal end, a distal end, at least one open channel formed on the exterior surface of said body to provide fluid communication between said proximal end and said distal end and a central lumen open at the proximal and distal ends of the stent for the passage of a guide wire, immobilizing the stent at the target site by using an anti-migration device extending from at least one end of the elongated body, and withdrawing the guide wire.

Disclosed herein, but not claimed, is also a method of removing a shell that holds the anti-migration device tucked, and deploying the anti-migration device. The method may further comprise using the anti-migration device extending from at least one end of the elongated body to push against a vessel, duct or lumen at said target site.

### EXAMPLES

### EXAMPLE 1: Endoscopic Retrograde Cholangio-Pancreatography (ERCP)

In a subject in need thereof, ECRP is a procedure performed to diagnose and treat diseases of the gallbladder, bile system, pancreas and liver.

An endoscope is passed though the mouth of the subject down through the stomach and into the duodenum, where the location of the entry of the bile duct into the small intestine is identified. The scope has a working channel (WC) through which a cannula (catheter) is fed and a practitioner "cannulates" the bile duct (to introduce the cannula into the bile duct). A guidewire is sent through the center lumen of the cannula and is passed thru the bile duct and into the liver. The cannula is removed and a sphincterotome is introduced. A practitioner cuts the papilla (the sphincter into bile duct) with the sphincterotome and the sphincterotome is withdrawn. An absorbable polymer stent as described herein is placed over the guide wire and pushed into the bile duct with a pushing catheter, allowing proper drainage through the bile duct. Depending upon the need of the subject, the stent is made of a fast absorbing polymer, medium-absorbing polymer or slow-absorbing polymer. The pushing catheter and guidewire are withdrawn from the subject.

### EXAMPLE 2: Simulated Flow Test

The setup for the simulated flow test involves a water container that is filled with tap water and connected with 6x9 mm silicone tube. The tube is further connected with a Y connector where one of the openings is connected to a pressure measurement device and another opening is attached to the silicone tube integrated into an endoscope/bile duct model, certified by physicians. The Y connector joint will be secured with cable ties against leakage. At the end of the silicone tube, a one-way stopcock should be attached for controlling water flow (on/off). Following the above setup, every stent is tested individually and inserted into the tube and positioned with help of a pushing catheter. Once the stent is positioned, stricture is made on the silicone tube by tightening cable ties around the stent, creating stricture of similar diameter as that of the stent. A pressure measurement device should set to 0 mbar before starting the test. Once the setup is secured, water should be released carefully from big water container and the flow will be adjusted till pressure measurement device displays 19 mbar. This value is calculated and derived from 20 cm H20 (I). As soon as the value sets to 19 mbar (I), 70 ml (II) of water will be collected in a measuring beaker and time for collecting will be counted with stop watch. The values considered in this protocol such as 70 ml and 19 mbar are understood from following references (these values are considered as worst case scenario): Csendes et al 1988 - Common Bile Duct Pressure in Patients With Common Bile Duct Stones With or Without Acute Suppurative Cholangitis states that common duct pressure values above 20 cm FhO is the maximal values of normal; and Dennison & Farrell 2016- Pass PCCN- 6. The G1 System states that Gallbladder stores bile and has a storage capacity of 50 to 70 ml. After every test, a beaker should be cleaned with a cloth to avoid reading errors/irregularities. The above mentioned procedure is followed for every single stent tested.

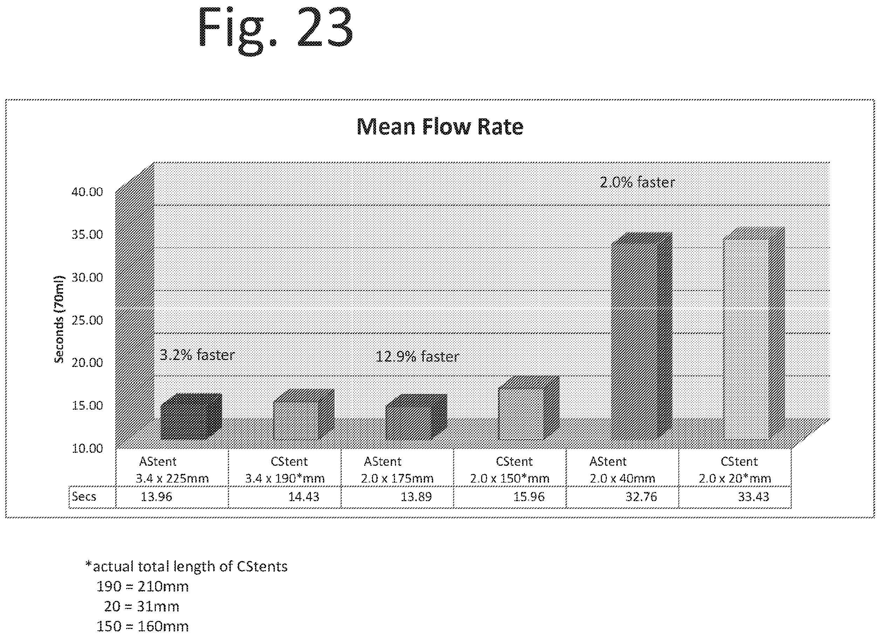

FIG. 23 shows the results of simulated flow model comparing the stent of the present application ("AStent") with some commercially available biliary and pancreatic stent (the control stents or "CStent"). The stent of the present application has a double-channel helical twist on the exterior surface of the stent body. The control stents do not have such a surface feature. In the simulated flow test, the stent of the present application ("AStent" in the figure) has a higher flow rate than the control stents ("CStent" in the figure) for all the comparisons shown (higher flow rate means less time needed to drainage the defined worst- case amount of simulated bile fluid). In particular, the 2.0 x 175 mm stent of the present application has a 12.9% faster mean flow rate than the comparable control stent, as used herein, the notion 2.0 x 175 mm, indicates a diameter of 2.0 mm and a length of 175 mm. A total of two test samples were tested for the stent sizes described. Each stent was tested five times for a total of ten data points per stent based on the recommendation in ASTM F2081.

### EXAMPLE 3: Simulated Migration Resistance Test

An endoscope model/biliary model is laid horizontally in a water bath at 37°C. A PTFE tube shall be fitted into model acting as bile duct. The stent is placed at a starting point of the tube and the end point of the tube will face towards a horizontal Zwick machine and load cell. A nylon string is inserted through the inner lumen of the stent and attached to the load cell of machine. The nylon string is fitted with a hook that helps to pull the stent through tube. Once the setup is secured, a protocol is created in the Zwick system, defining all parameters such as speed (200mm/min) and travel distance (120 mm). After each stent is tested, the anti-migration struts are visually inspected. Any broken or damaged anti-migration struts observed shall be photographed and documented in the report. As the stent is pulled through the model, the load cell measures the friction force and the force values are automatically recorded in the Zwick system. The peak friction forces of each stent tested are documented in the report. Each stent was tested five times for a total of ten data points per stent based on the recommendation in ASTM F2081.

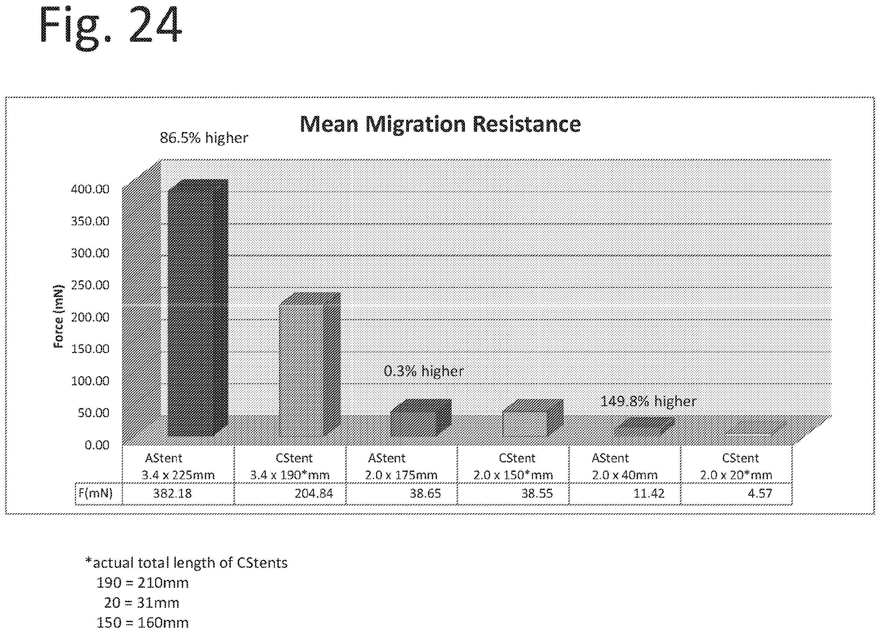

FIG. 24 shows the results of a simulated migration resistance test comparing the stent of the present application to control biliary and pancreatic stents. The stent of the present application has higher mean migration resistance compared to the control stents for all the comparisons shown. In particular, the 2.0 x 40 mm stent of the present application has 149.8% higher mean migration resistance than the control stent.

### EXAMPLE 4: Crush Resistance Test

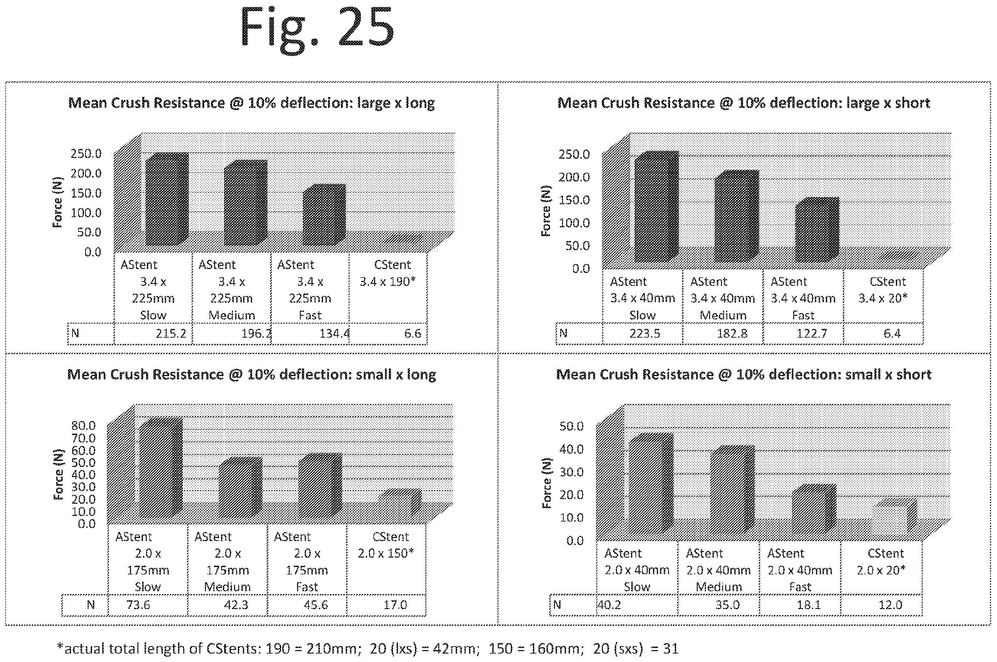

FIG. 25 shows the results of crush resistance tests comparing the stent of the present application to control biliary and pancreatic stents. The tests are carried out using a 6mm flat probe on a tensile test machine. The stent is laying on a flat plate and the probe comes down on the stent from the top. The stent is compressed 10% of the diameter of the stent and then peak force is measured, e.g., 2.0 mm stent: 10% deflection= 0.2 mm deflection; 2.6 mm stent: 10% deflection= 0.26 mm deflection; 3.4 mm stent: 10% deflection= 0.34 mm deflection,

Stents of the present application with different degradation profiles were tested (shown as "slow", "medium" and "fast" in the figure). In each case, the stent of the present application showed superior mean crush resistance compared to the control stents. In particular, for each comparison shown, the stents of the present application which have a slow degradation profile have a significantly elevated level of crush resistance in comparison to the control stents.

### EXAMPLE 5: Animal Tests

The stents of the present application have been tested successfully in animal studies. A study was designed to evaluate the feasibility of the fully biodegradable plastic biliary stent in a clinically relevant porcine common bile duct model determining deliverability, safety, and the biological reaction of the bile duct.

Two animals were treated with stents in the biliary ducts. Animals were clinically evaluated throughout the study period. After 24 day follow-up angiography and endoscopy, animals were then taken off study and saved for future use. Swine are an established model for physiological monitoring and bile duct studies. The size of porcine bile ducts can approximate that of a human and will accommodate the necessary instrumentation. Animals were fasted 72 hours, received EnerCal for nutritional supplement, prior to Day 0 procedures. On Day 0, animals were given an injection of Telazol for sedation and then transported to the prep room once sufficiently sedate. Animals were administered 5% Isoflurane via nose cone for anesthetic induction then intubated. General anesthesia was maintained with Isoflurane in oxygen, administered via a standard rebreathing system.

An endoscope was introduced orally and then traversed through the esophagus, stomach and into the duodenum. The ampullary region was then identified and the biliary duct was cannulated with a catheter. Angiograms were recorded using non-ionic contrast to help visualize the biliary ducts. Stents were implanted in the bile ducts of each animal by advancing over wire guide to target site. One animal (P272) had 4 stents implanted in the bile ducts with the possibility of one being unsuccessful and falling out and the other animal (P273) had 3 stents implanted in the pancreatic and bile ducts. On second implant animal P273 had 3 stents implanted. Stents were either placed parallel or lined up in a row. The number, order and location of stent implant was at the discretion of operator and was chosen based on accessibility. An angiogram was recorded to verify placement. Following post-stent angiography/fluoroscopy in chronic animals, wire guides and catheters were withdrawn along with the endoscope. The anesthesia was discontinued and the animal allowed to recover in a raised floor pen.

For interim/follow-up procedures, at 72 hours, 6, 14, and 24 days following implantation, animals were prepped in the same manner as for implant (with a 48 hour fasting). On second implant for P273 animal was fasted 24 hours prior to 7, 14, and 17 day follow-ups. Endoscope was advanced to duodenum to visualize the stents. Angiograms of the bile ducts were taken using non-ionic contrast agent. For interim procedures the endoscope was withdrawn. The anesthesia was discontinued and the animal allowed to recover in a raised floor pen. For endpoint procedures (Day 24), anesthesia was discontinued and the animal allowed to recover in a raised floor pen. This study was considered complete as no devices were visible and animals were then used in additional studies (P272) or reimplanted (P273). At the endpoint (Day 17) of second implant, for P273, and completion of additional studies, for P272, animals received a 10000u IV heparin bolus (P272 only) and were sacrificed and necropsied and bile ducts were sent for histopathology.

Both animals had no significant clinical observations during the in-life period. Recovery after every procedure was uneventful and animal returned to normal within 24 hours. In general, the follow-up procedure consisting of final angiograms to visualize stents in bile ducts. No abnormal findings were observed during the follow-up procedure in P272 or P273. After the final images were captured, animals were taken off anesthesia and allowed to recover. They were then used to test other devices at a later date (P272) or reimplanted (P273). Once those studies were complete a 10,000 unit bolus of Heparin was administered and allowed to circulate (P272 only) then the animals were euthanized with 40mEq of Potassium chloride IV while under a deep plane of anesthesia. Death was verified by a lack of vital signs. Animals were then transported to necropsy for vessel harvesting procedures. Pancreas and bile ducts were sent for histopathology.

Histopathology involved taking the formalin fixed bile duct from the gall bladder to the duodenum plus surrounding tissues including pancreas for histopathology. The bile duct was sectioned every 1-1.5 cm from the gall bladder to the duodenum including the duodenal papilla. A distinct pancreatic duct was not identified on gross examination. Slides were processed for paraffin histopathology using routine techniques, cut at 5 microns, and stained with hematoxylin and eosin stain. The outcome of the study showed that, after gross examination, the liver, bile duct, and duodenum were normal with no important changes. Microscopically, no lesions were identified in the bile duct at any level. Liver and duodenum were similarly normal. On gross examination, the pancreas was normal. A distinct pancreatic duct was not identified. Loose tissues between the pancreas and duodenum were examined grossly and microscopically for pancreatic ducts, but none were found. The pancreatic duct was found grossly and microscopically within pancreatic lobules. No important changes other than early autolysis were found. Three small (up to 4 mm) areas of chronic inflammation (eosinophilic granulomas) were present in the peripancreatic connective tissue near the duodenum. The pancreatic ducts were not identified within the eosinophilic granulomas.

### EXAMPLE 6: Human Tests

The stents of the present application have been placed in a study involving twenty-four patients. The study assessed the safety and efficacy of the plastic biliary stent in patients with benign and malign biliary strictures and who suffer under jaundice and pruritus. Since this device is deemed to drain obstructed biliary or pancreatic ducts the aim is to improve bile flow in patients suffering from benign and malignant biliary strictures. The study had first and second endpoints. The first endpoint concerned safety, which the study defined in terms of complications seen in PEP (Post-Endoscopic Perforation) like bleeding, perforation (bile/duodenum), misplacement, migration, bile occlusion, duct abrasion, duodenal abrasion, cholangitis, severe pain and all-death. The second endpoint concerned efficacy, which the study defined in terms of the following markers of procedural success: stent loadability; trackability over guide wire; pushability with push-catheter; flexibility; force required to implant device; visualization by fluoroscopy; stent reposition when required; deployment accuracy; device deployment time.

The patient cohort consisted of twenty-four patients (average age 54.5 years, female 42%, male 58%); the variety of patient conditions included malignant biliary structure (3), benign biliary structure (21), cholocystitis (2), cholelethiasis (2), choledocholithiasis (11), cholangitis (6), pancreatitis (3), ampullary cancer (1), pancreatic head cancer (2). The study proved successful procedurally for all patients in terms of stent loadability (clinical ratings: 78% excellent, 17% good, 4% poor); stent trackability over guide wire (clinical ratings: 87% excellent, 9% good, 4% fair); stent pushability with push catheter (clinical ratings: 74% excellent, 13% good, 9% fair, 4% poor; force required to implant device (clinical ratings: 57% excellent, 21% good, 13% fair, 9% poor); stent flexibility (clinical ratings: 57% excellent, 4% good, 26% fair, 13% poor); visibility by fluoroscopy (clinical ratings: 88% excellent, 14% fair); stent repositioning when required (clinical ratings: 86% excellent, 14% fair); deployment accuracy (clinical ratings: 84% excellent, 8% good, 8% fair); and device deployment time (clinical ratings: 76% excellent, 11% good, 9% fair, 4% poor). Procedural complications were experienced by five patients who had mild complications during the procedure assessed as bleeding after sphincteroplasty (3), supraventricular tachycardia (1) and hyperamylasemia (1). Throughout the twelve month follow-up period only one event occurred which caused particular treatment (this event emerged at day one after index-procedure and did not prolong the patient's hospital stay). In this instance, the patient developed elevated serum amylase without epigastric pain at day one after procedure, however, this was a very mild case and vanished spontaneously in twenty-four hours.

The stents of the present application have also been successfully tested in a study of prophylactic stenting in an anastomotic site in liver transplants in three patients.

In another study to show equivalence to standard plastic biliary stents, an elderly man in his early to mid to late sixties presented with repeat jaundice due to repeat stone obstruction in the common bile duct. The patient had been treated numerous times in the past for common bile duct obstruction due to stone formation. It was decided to cannulate the patient's common bile duct, perform stone removal using a standard balloon and repeat dilation of the duct and the papilla, and then stent using a fast absorbing biodegradable stent. The benefit of the biodegradable embodiment herein is that the follow up procedure to remove the stent can be eliminated as well as posterior wall irritation can be avoided through degradation. The papilla was distended and dilated and it was initially feared that one stent would dislodge and might not be adequate for the lesions. This fast absorbing device was placed using a standard guide wire (in certain embodiments, a super stiff wire may be advantageous for placing the stent into the duct). A 120 mm long, 3.4 mm diameter stent after duct dilation for stone removal was delivered without incident over the wire while manually manipulating the proximal antimigration as it was pushed into the scope. The stent entered the duct easily and the endoscope was positioned at the entrance of the papilla with limited distance and minor use of the scopes elevator to insure easy access. The stent entered the duct much like a metal stent when used.

On-going studies are being performed. For example, a study in a fifty year old female with a benign biliary structure, in which after pre-dilation a 3.4mm OD x 120 cam long, slow absorbing plastic biliary stent has been implanted. In another example, after thirty days post-procedure, the physician case report indicated the patient was asymptomatic and had no complications (no x-ray or fluoro imaging was required).

In another instance, Y-shape stents can be used to open the fallopian tubes where the distal Y-shape flaps/tails are in the ovaries and the proximal Y-shape flaps/tails transverse the fallopian tubes until the stent reaches the uterus. In another embodiment, the stent is a conduit to connect any existing organ that has a tube that connects to another organ for the passage of fluid, for example, connecting a kidney to a bladder.

The above description is for the purpose of teaching the person of ordinary skill in the art how to practice the present application, and it is not intended to detail all those obvious modifications and variations of it which will become apparent to the skilled worker upon reading the description. It is intended, however, that all such obvious modifications and variations be included within the scope of the present application, which is defined by the following claims. The claims are intended to cover the claimed components and steps in any sequence which is effective to meet the objectives there intended, unless the context specifically indicates the contrary.

## Claims

1. A stent (100, 400, 500, 1000, 1500, 1900) comprising:
an elongated body (10) composed of a biodegradable material having a proximal end (12, 1201), a distal end (14, 1203), at least one open spiral channel formed on the exterior surface of said body to provide fluid communication between said proximal end (12, 1201) and said distal end (14, 1203),
a central lumen (20, 1205, 1907) open at the proximal (12, 1201) and distal ends (14, 1203) of the stent for the passage of a guide wire (24), and
wherein said stent further comprises at least two anti-migration tails (1505, 1910) extending beyond at least one end of the elongated body (10),
wherein the at least two anti-migration tails (1505, 1910) extending from at least one end of the elongated body (10) form a Y-shape with the elongated body (10), and
wherein said anti-migration tails (1505, 1910) are flat-shaped at their ends.

2. The stent of Claim 1, wherein the at least two anti-migration tails (1505, 1910) extending from at least one end of the elongated body (10) are different in length.

3. The stent of Claim 1, wherein the at least two anti-migration tails (1505, 1910) extending from at least one end of the elongated body (10) are different in thickness.

4. The stent of Claim 1, wherein the at least two anti-migration tails (1505, 1910) extending from at least one end of the elongated body (10) are made of different material than the elongated body (10).

5. The stent of Claim 1, wherein at least one of: length, thickness, and flexibility of the at least two anti-migration tails (1505, 1910) are determined based on expected dimensions of a target site for stent placement relative to dimensions of the stent (100, 400, 500, 1000, 1500, 1900).

6. The stent of Claim 1, wherein a tip of the at least two anti-migration tails (1505, 1910) extending from at least one end of the elongated body (10) is radiused.

7. The stent of Claim 1, further comprising a shell that holds the at least two anti-migration tails (1505, 1910) tucked.

8. The stent of Claim 1, wherein the at least two anti-migration tails (1505, 1910) extending from at least one end of the elongated body (10) is configured to be held flush with the surface of the stent (100, 400, 500, 1000, 1500, 1900) prior to and during deployment and to fold out following placement at the target site.

## Patentansprüche

1. Stent (100, 400, 500, 1000, 1500, 1900), umfassend:
einen länglichen Körper (10), der aus einem biologisch abbaubaren Material zusammengesetzt ist und ein proximales Ende (12, 1201), ein distales Ende (14, 1203) und mindestens einen offenen Spiralkanal aufweist, der an der Außenfläche des Körpers ausgebildet ist, um eine Fluidverbindung zwischen dem proximalen Ende (12, 1201) und dem distalen Ende (14, 1203) bereitzustellen,
ein zentrales Lumen (20, 1205, 1907), das an dem proximalen (12, 1201) und distalen Ende (14, 1203) des Stents für den Durchgang eines Führungsdrahtes (24) offen ist, und
wobei der Stent ferner mindestens zwei Migrationsschutzendstücke (1505, 1910) umfasst, die sich über mindestens ein Ende des länglichen Körpers (10) hinaus erstrecken,
wobei die mindestens zwei sich von mindestens einem Ende des länglichen Körpers (10) erstreckenden Migrationsschutzendstücke (1505, 1910) eine Y-Form mit dem länglichen Körper (10) bilden und
wobei die Migrationsschutzendstücke (1505, 1910) an ihren Enden flach geformt sind.

2. Stent nach Anspruch 1, wobei die mindestens zwei sich von mindestens einem Ende des länglichen Körpers (10) erstreckenden Migrationsschutzendstücke (1505, 1910) unterschiedliche Längen aufweisen.

3. Stent nach Anspruch 1, wobei die mindestens zwei sich von mindestens einem Ende des länglichen Körpers (10) erstreckenden Migrationsschutzendstücke (1505, 1910) unterschiedliche Dicken aufweisen.

4. Stent nach Anspruch 1, wobei die mindestens zwei sich von mindestens einem Ende des länglichen Körpers (10) erstreckenden Migrationsschutzendstücke (1505, 1910) aus einem anderen Material als der längliche Körper (10) hergestellt sind.

5. Stent nach Anspruch 1, wobei mindestens eines von: Länge, Dicke und Flexibilität der mindestens zwei Migrationsschutzendstücke (1505, 1910) basierend auf erwarteten Abmessungen einer Zielstelle für die Stentplatzierung relativ zu Abmessungen des Stents (100, 400, 500, 1000, 1500, 1900) bestimmt wird.

6. Stent nach Anspruch 1, wobei eine Spitze der mindestens zwei sich von mindestens einem Ende des länglichen Körpers (10) erstreckenden Migrationsschutzendstücke (1505, 1910) abgerundet ist.

7. Stent nach Anspruch 1, ferner umfassend einen Mantel, der die mindestens zwei Migrationsschutzendstücke (1505, 1910) zusammenhält.

8. Stent nach Anspruch 1, wobei die mindestens zwei sich von mindestens einem Ende des länglichen Körpers (10) erstreckenden Migrationsschutzendstücke (1505, 1910) dazu konfiguriert sind, vor und während des Einsatzes bündig mit der Oberfläche des Stents (100, 400, 500, 1000, 1500, 1900) gehalten zu werden und sich nach der Platzierung an der Zielstelle zu entfalten.

## Revendications

1. Endoprothèse (100, 400, 500, 1000, 1500, 1900) comprenant :
un corps allongé (10) composé d'un matériau biodégradable présentant une extrémité proximale (12, 1201), une extrémité distale (14, 1203), au moins un canal spiralé ouvert formé sur la surface extérieure dudit corps pour assurer une communication fluidique entre ladite extrémité proximale (12, 1201) et ladite extrémité distale (14, 1203),
une lumière centrale (20, 1205, 1907) ouverte au niveau des extrémités proximale (12, 1201) et distale (14, 1203) de l'endoprothèse pour le passage d'un fil-guide (24), et
dans laquelle ladite endoprothèse comprend également au moins deux ailettes anti-migration (1505, 1910) se prolongeant au-delà d'au moins une extrémité du corps allongé (10),
dans laquelle les au moins deux ailettes anti-migration (1505, 1910) se prolongeant à partir d'au moins une extrémité du corps allongé (10) forment une forme en Y avec le corps allongé (10), et
dans laquelle lesdites ailettes anti-migration (1505, 1910) sont de forme plate à leurs extrémités.

2. Endoprothèse selon la revendication 1, dans laquelle les au moins deux ailettes anti-migration (1505, 1910) se prolongeant à partir d'au moins une extrémité du corps allongé (10) présentent des longueurs différentes.

3. Endoprothèse selon la revendication 1, dans laquelle les au moins deux ailettes anti-migration (1505, 1910) se prolongeant à partir d'au moins une extrémité du corps allongé (10) présentent des épaisseurs différentes.

4. Endoprothèse selon la revendication 1, dans laquelle les au moins deux ailettes anti-migration (1505, 1910) se prolongeant à partir d'au moins une extrémité du corps allongé (10) sont constituées d'un matériau différent de celui du corps allongé (10) .

5. Endoprothèse selon la revendication 1, dans laquelle au moins l'une : de la longueur, de l'épaisseur et de la flexibilité des au moins deux ailettes anti-migration (1505, 1910) sont déterminées sur la base des dimensions attendues d'un site cible pour la mise en place de l'endoprothèse par rapport aux dimensions de l'endoprothèse (100, 400, 500, 1000, 1500, 1900).

6. Endoprothèse selon la revendication 1, dans laquelle une extrémité des au moins deux ailettes anti-migration (1505, 1910) se prolongeant à partir d'au moins une extrémité du corps allongé (10) est arrondie.

7. Endoprothèse selon la revendication 1, comprenant également une coque qui maintient repliées les au moins deux ailettes anti-migration (1505, 1910).

8. Endoprothèse selon la revendication 1, dans laquelle les au moins deux ailettes anti-migration (1505, 1910) se prolongeant à partir d'au moins une extrémité du corps allongé (10) sont configurées pour être maintenues à fleur de la surface de l'endoprothèse (100, 400, 500, 1000, 1500, 1900) avant et pendant le déploiement, et pour se déployer après la mise en place sur le site cible.
